# EUROPÄISCHE PATENTSCHRIFT

(11) **EP 2 062 868 B1**
(45) Veröffentlichungstag und Bekanntmachung des Hinweises auf die Patenterteilung: **24.02.2016**
(21) Anmeldenummer: 08019730.4
(22) Anmeldetag: 12.11.2008
(51) Int. Cl.: C07C 68/08, C07C 69/96, B01D 3/14, C07C 68/06

(54) **Verfahren zur Reinigung von Diarylcarbonaten**
Process for purifying diaryl carbonates
Procédé de purification de carbonates de diaryle

(30) Priorität: 20.11.2007 DE 102007055266
(43) Veröffentlichungstag der Anmeldung: 27.05.2009
(73) Patentinhaber: Covestro Deutschland AG, 51373 Leverkusen (DE)
(72) Erfinder: Ooms, Pieter, Dr., 47800 Krefeld (DE); Rechner, Johann, Dr., 47906 Kempen (DE); Böhm, Matthias, 51373 Leverkusen (DE); Düx, Andre, 50321 Brühl (DE); Hallenberger, Kaspar, 51375 Leverkusen (DE); Ronge, Georg, Dr., 40549 Düsseldorf (DE); Vanden Eynde, Johan, 9052 Zwijnaarde Gent (BE)
(74) Vertreter: Levpat

(56) Entgegenhaltungen:
- EP-A- 0 784 048
- EP-A- 1 803 704
- DE-A1- 3 302 525
- JP-A- 3 291 257

## Beschreibung

Gegenstand der vorliegenden Erfindung ist ein spezielles Verfahren zur Reinigung von Diarylcarbonaten.

Die Aufreinigung von Diarylcarbonat ist aufgrund der hohen Anforderungen an deren Reinheit für die Herstellung von qualitativ hochwertigen Polycarbonaten mittels Schmelzeumesterung von großer Bedeutung. Diarylcarbonate hergestellt aus Umesterung einer aromatischen Hydroxyverbindung mit einem Alkylcarbonat können sowohl hochsiedende als auch mittelsiedende Nebenkomponenten sowie Katalysatorreste als Verunreinigungen enthalten. Hochsiedende Komponenten - oft auch als Schwersieder bezeichnet - im Sinne dieses Herstellverfahrens sind solche, deren Siedepunkt oberhalb desjenigen des Diarylcarbonats liegen. Mittelsiedende Komponenten - oft auch als Zwischensieder bezeichnet - im Sinne dieses Herstellverfahrens sind solche, deren Siedepunkt zwischen dem des Diarylcarbonats und dem des während der Herstellung des Diarylcarbonats als Nebenprodukt gebildeten Alkylarylcarbonats liegt. All diese Verunreinigungen führen zu erheblicher qualitativer Beeinträchtigung der herzustellenden Polycarbonate und müssen vor der Weiterverarbeitung der Diarylcarbonate durch entsprechende Aufreinigung entfernt werden.

In der WO-A 2004/113264 wird ein Verfahren zur Rückgewinnung eines Produktstromes aus einem Abfallstrom bei der Herstellung von Diarylcarbonat beschrieben. Dabei wird auch eine Aufarbeitung des Diarylcarbonats, welches in einer aus drei Reaktionskolonnen bestehenden Reaktion hergestellt wurde, beschrieben, die in drei Stufen bestehend aus Katalysatorabtrennung, Leichtsieder- und Hochsiederabtrennung durchgeführt wird. Sowohl in ersten als auch im letztgenannten Schritt wird das Diarylcarbonat als Kopfprodukt abgetrennt. Das beschriebene Verfahren ist nicht nur apparativ äußerst aufwändig, sondern auch aufgrund der Abtrennung des Diarylcarbonats sowohl in der ersten als auch in der dritten Stufe als Kopfprodukt energetisch ungünstig. Zudem ist die Qualität des so hergestellten Diarylcarbonats mit 99.5 Gew.-% sehr schlecht und für die Herstellung von qualitativ hochwertigem Polycarbonat nicht geeignet.

In der EP-A 1 760 069 wird ein Verfahren zur Herstellung von Diarylcarbonaten beschrieben, bei dem nach der Umesterung einer aromatischen Hydroxyverbindung mit einem Alkylcarbonat ein Gemisch erhalten wird, dass neben Diarylcarbonat unter anderem auch einen aromatischen Carbonatether als Verunreinigung enthält. Letzterer wird in einem folgenden Schritt abgetrennt, um ein hochreines Diarylcarbonat zu erhalten. Die deutlich aufwändigere Abtrennung hochsiedender Nebenkomponenten, die zu qualitativer Beeinträchtigung der Zielprodukte (Polycarbonate) führen würden, wird jedoch nicht erwähnt.

In EP 1 783 112 A1, EP 1 787 977 A1, EP 1 801 095 A1 und EP 1 787 976 A1 wird ein aus mindestens zwei Stufen bestehendes Verfahren zur Aufreinigung von Diarylcarbonat, insbesondere Diphenylcarbonat, beschrieben, bei dem nach der Reaktion zunächst die hochsiedenden Komponenten und der Katalysator abgetrennt werden. Das bei dieser Hochsiederabtrennung anfallende Destillat wird in einer zweiten Destillationskolonne in drei Fraktionen aufgetrennt, wobei man Diarylcarbonat im Seitenstrom entnimmt. Nachteilig an diesem Verfahren ist jedoch, dass die Abtrennung der hochsiedenden Komponenten im ersten Schritt erfolgt, da dies energetisch ungünstig ist.

In der EP-A 784 048 wird ein Verfahren zur Reinigung von Diarylcarbonaten beschrieben, bei der eine Destillation bei einer Sumpftemperatur größer 150 °C durchgeführt wird und die Produktentnahme des gereinigten Diarylcarbonats im Seitenstrom der Kolonne erfolgt. Die Herstellung der zu reinigenden Diarylcarbonate kann entweder durch Umsetzung von Carbonylhalogeniden mit aromatischen Hydroxyverbindungen oder durch Umesterung aromatischer Hydroxyverbindungen mit Dimethylcarbonat erfolgen. Zwar wird durch das beschriebene Verfahren ermöglicht, Diarylcarbonate weitestgehend von Schwersiedern, wie beispielsweise Salicylsäurephenylester, zu befreien, jedoch ist von der Gegenwart störender Katalysatorreste oder Zwischensiedern oder einer Möglichkeit zu deren Entfernung an keiner Stelle die Rede. Insbesondere die Gegenwart von nennenswerten Mengen an Katalysatorresten kann jedoch eine destillative Aufreinigung im Ergebnis deutlich beeinflussen, da hierdurch die Nebenreaktion von Diarylcarbonat zum Salicylsäurephenylester beschleunigt und damit dessen Anreicherung im Sumpf der Kolonne gefördert wird. Daher ist das in EP-A 784 048 beschriebene Verfahren für eine solche Reinigung in Gegenwart von Katalysatorresten und/oder Zwischensiedern nicht ohne weiteres geeignet.

Es bestand demnach weiterhin Bedarf an einem Verfahren zur Aufreinigung von Diarylcarbonaten bei dem mit möglichst einfacher apparativer und günstiger energetischer Ausführung Katalysatorreste und hochsiedende Nebenkomponenten sowie gegebenenfalls auch mittelsiedende Nebenkomponenten als Verunreinigungen entfernt werden können.

Die Aufgabe, die der vorliegenden Erfindung zugrunde lag, bestand darin, ein solches Verfahren bereitzustellen.

Überraschend wurde nun gefunden, dass die destillative Aufreinigung von Diarylcarbonaten auch bei geringem apparativen und energetischen Aufwand durchgeführt werden kann, wenn die zu reinigenden Diarylcarbonate Katalysatorreste und hochsiedende Nebenkomponenten sowie gegebenenfalls auch mittelsiedende Nebenkomponenten als Verunreinigungen enthalten. Dabei kann bevorzugt sogar die bei der Destillation anfallende Abwärme energetisch genutzt werden.

Gegenstand der vorliegenden Erfindung ist ein Verfahren zur Reinigung von Diarylcarbonaten in wenigstens einer Destillationskolonne enthaltend wenigstens einen Verstärkungsteil im oberen Teil der Kolonne und wenigstens einen Abtriebsteil im unteren Teil der Kolonne, wobei
- das zu reinigende Diarylcarbonat durch Umesterung aus wenigstens einem Dialkylcarbonat und wenigstens einer aromatischen Hydroxyverbindung in Gegenwart wenigstens eines Umesterungskatalysator hergestellt wurde und Katalysator aus der Herstellung des Diarylcarbonats als Verunreinigung enthält, und
- das gereinigte Diarylcarbonat im Seitenstrom der ersten Destillationkolonne entnommen wird;
dadurch gekennzeichnet, dass es sich bei der ersten Destillationskollonne um eine Trennwandkolonne handelt.

Im Rahmen der Erfindung zu reinigende Diarylcarbonate sind bevorzugt solche der allgemeinen 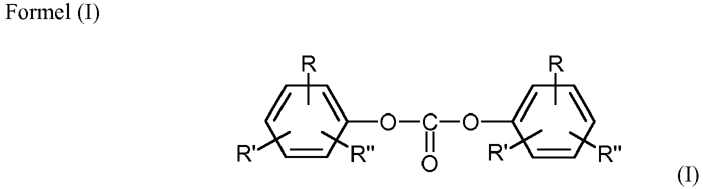 wobei R, R' und R" unabhängig voneinander H, lineares oder verzweigtes, gegebenenfalls substituiertes C₁-C₃₄-Alkyl, bevorzugt C₁-C₆-Alkyl, besonders bevorzugt C₁-C₄-Alkyl, C₁-C₃₄-Alkoxy, bevorzugt C₁-C₆-Alkoxy, besonders bevorzugt C₁-C₄-Alkoxy, C₅-C₃₄-Cycloalkyl, C₇-C₃₄-Alkylaryl, C₆-C₃₄-Aryl oder einen Halogenrest, bevorzugt einen Chlorrest darstellen und R, R' und R" auf beiden Seiten der Formel (I) gleich oder verschieden sein können. R kann auch -COO-R"' bedeuten, wobei R'" H, gegebenenfalls verzweigtes C₁-C₃₄ Alkyl, bevorzugt C₁-C₆-Alkyl, besonders bevorzugt C₁-C₄-Alkyl, C₁-C₃₄-Alkoxy, bevorzugt C₁-C₆-Alkoxy, besonders bevorzugt C₁-C₄-Alkoxy, C₅-C₃₄-Cycloalkyl, C₇-C₃₄-Alkylaryl oder C₆-C₃₄-Aryl sein kann. Bevorzugt sind R, R' und R" auf beiden Seiten der Formel (I) gleich. Ganz besonders bevorzugt stehen R, R' und R" für H.

Diarylcarbonate der allgemeinen Formel (I) sind beispielsweise: Diphenylcarbonat, Methylphenylphenyl-carbonate und Di-(methylphenyl)-carbonate, auch als Gemisch, wobei die Stellung der Methylgruppe an den Phenylringen beliebig sein kann, sowie Dimethylphenyl-phenyl-carbonate und Di-(dimethylphenyl)-carbonate, auch als Gemisch, wobei die Stellung der Methylgruppen an den Phenylringen beliebig sein kann, Chlorphenyl-phenyl-carbonate und Di-(chlorphenyl)-carbonate, wobei die Stellung der Methylgruppe an den Phenylringen beliebig sein kann, 4-Ethylphenyl-phenyl-carbonat, Di-(4-ethylphenyl)-carbonat, 4-n-Propylphenyl-phenyl-carbonat, Di-(4-n-propylphenyl)-carbonat, 4-iso-Propylphenyl-phenyl-carbonat, Di-(4-iso-propylphenyl)-carbonat, 4-n-Butylphenyl-phenyl-carbonat, Di-(4-n-butylphenyl)-carbonat, 4-iso-Butylphenyl-phenyl-carbonat, Di-(4-iso-butylphenyl)-carbonat, 4-tert-Butylphenyl-phenyl-carbonat, Di-(4-tert-butylphenyl)-carbonat, 4-n-Pentylphenyl-phenyl-carbonat, Di-(4-n-pentylphenyl)-carbonat, 4-n-Hexylphenyl-phenyl-carbonat, Di-(4-n-hexylphenyl)-carbonat, 4-iso-Octylphenyl-phenyl-carbonat, Di-(4-iso-octylphenyl)-carbonat,4-n-Nonylphenyl-phenyl-carbonat, Di-(4-n-nonylphenyl)-carbonat, 4-Cyclohexylphenyl-phenyl-carbonat, Di-(4-cyclohexylphenyl)-carbonat, 4-(1-Methyl-1-phenylethyl)-phenyl-phenyl-carbonat, Di-[4-(1-methyl-1-phenylethyl)-phenyl]-carbonat, Biphenyl-4-yl-phenyl-carbonat, Di-(biphenyl-4-yl)-carbonat, (1-Naphthyl)-phenyl-carbonat, (2-Naphthyl)-phenyl-carbonat, Di-(1-naphthyl)-carbonat, Di-(2-naphthyl)-carbonat, 4-(1-Naphthyl)-phenyl-phenyl-carbonat, 4-(2-Naphthyl)-phenyl-phenyl-carbonat, Di-[4-(1-naphthyl)phenyl]-carbonat, Di-[4-(2-naphthyl)phenyl]-carbonat, 4-Phenoxyphenyl-phenyl-carbonat, Di-(4-phenoxyphenyl)-carbonat, 3-Pentadecylphenyl-phenyl-carbonat, Di-(3-pentadecylphenyl)-carbonat, 4-Tritylphenylphenyl-carbonat, Di-(4-tritylphenyl)-carbonat, Methylsalicylat-phenyl-carbonat, Di-(methylsalicylat)-carbonat, Ethylsalicylat-phenyl-carbonat, Di-(ethylsalicylat)-carbonat, n-Propylsalicylat-phenyl-carbonat, Di-(n-propylsalicylat)-carbonat, iso-Propylsalicylat-phenyl-carbonat, Di-(iso-propylsalicylat)-carbonat, n-Butylsalicylat-phenyl-carbonat, Di-(n-butylsalicylat)-carbonat, iso-Butylsalicylat-phenyl-carbonat, Di-(iso-butylsalicylat)-carbonat, tert-Butylsalicylat-phenyl-carbonat, Di-(tert-butylsalicylat)-carbonat, Di-(phenylsalicylat)-carbonat und Di-(benzylsalicylat)-carbonat.

Bevorzugte Diarylcarbonate sind: Diphenylcarbonat, 4-tert-Butylphenyl-phenyl-carbonat, Di-(4-tert-butylphenyl)-carbonat, Biphenyl-4-yl-phenyl-carbonat, Di-(biphenyl-4-yl)-carbonat, 4-(1-Methyl-1-phenylethyl)-phenyl-phenyl-carbonat und Di-[4-(1-methyl-1-phenylethyl)-phenyl]-carbonat.

Besonders bevorzugt ist Diphenylcarbonat.

Dialkylcarbonate im Rahmen der Erfindung sind solche der Formel (II) 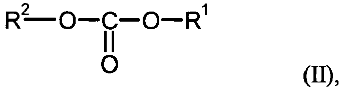 wobei R¹ und R² unabhängig voneinander für lineares oder verzweigtes, gegebenenfalls substituiertes C₁-C₃₄-Alkyl, bevorzugt für C₁-C₆-Alkyl, besonders bevorzugt für C₁-C₄-Alkyl stehen. Dabei können R¹ und R² gleich oder verschieden sein. Bevorzugt sind R¹ und R² gleich.

Bevorzugte Dialkylcarbonate sind Dimethylcarbonat, Diethylcarbonat, Di(n-propyl)carbonat, Di(iso-propyl)carbonat, Di(n-butyl)carbonat, Di(sec-butyl)carbonat, Di(tert-butyl)carbonat oder Dihexylcarbonat. Besonders bevorzugt sind Dimethylcarbonat oder Diethylcarbonat. Ganz besonders bevorzugt ist Dimethylcarbonat.
Aromatische Hydroxyverbindungen im Rahmen der Erfindung sind bevorzugt solche der allgemeinen Formel (III) 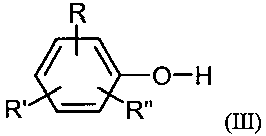 worin R, R' und R" unabhängig voneinander die für die allgemeine Formel (I) genannte Bedeutung haben können.

Solche aromatischen Hydroxyverbindungen sind beispielsweise: Phenol, o-, m- oder p-Kresol, auch als Gemisch der Kresole, Dimethylphenol, auch als Gemisch, wobei die Stellung der Methylgruppen am Phenolring beliebig sein kann, z.B. 2,4-, 2,6-, oder 3,4-Dimethylphenol, o-, m- oder p-Chlorphenol, o-, m- oder p-Ethylphenol, o-, m- oder p-n-Propylphenol, 4-iso-Propylphenol, 4-n-Butylphenol, 4-iso-Butylphenol, 4-tert-Butylphenol, 4-n-Pentylphenol, 4-n-Hexylphenol, 4-iso-Octylphenol, 4-n-Nonylphenol, o-, m- oder p-Methoxyphenol, 4-Cyclohexylphenol, 4-(1-Methyl-1-phenylethyl)-phenol, Biphenyl-4-ol, 1-Naphthol, 2-1-Naphthol, 4-(1-Naphthyl)phenol, 4-(2-Naphthyl)-phenol, 4-Phenoxyphenol, 3-Pentadecylphenol, 4-Tritylphenol, Methylsalicylsäure, Ethylsalicylsäure, n-Propylsalicylsäuret, iso-Propylsalicylsäure, n-Butylsalicylsäure, iso-Butylsalicylsäure, tert-Butylsalicylsäure, Phenylsalicylsäure und Benzylsalicylsäure.

Bevorzugte aromatischen Hydroxyverbindungen sind Phenol, 4-tert-Butylphenol, Biphenyl-4-ol und 4-(1-Methyl-1-phenylethyl)-phenol.

Besonders bevorzugt ist Phenol.

Sowohl die vorangehend genannten Dialkylcarbonate als auch die aromatischen Hydroxyverbindungen sind dem Fachmann bekannt und kommerziell erhältlich oder können nach dem Fachmann ebenfalls bekannten Verfahren hergestellt werden.

**C₁-C₄-Alkyl** steht im Rahmen der Erfindung beispielsweise für Methyl, Ethyl, n-Propyl, isoPropyl, n-Butyl, sec.-Butyl, tert.-Butyl, **C₁-C₆-Alkyl** darüber hinaus beispielsweise für n-Pentyl, 1-Methylbutyl, 2-Methylbutyl, 3-Methylbutyl, neo-Pentyl, 1-Ethylpropyl, n-Hexyl, 1,1-Dimethylpropyl, 1,2-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl oder 1-Ethyl-2-methylpropyl, **C₁-C₃₄-Alkyl** darüber hinaus beispielsweise für n-Heptyl und n-Octyl, n-Nonyl, n-Decyl, n-Dodecyl, n-Tridecyl, n-Tetradecyl, n-Hexadecyl oder n-Octadecyl. Gleiches gilt für den entsprechenden Alkylrest beispielsweise in Aralkyl- bzw. Alkylarylresten. Alkylenreste in den entsprechenden Hydroxyalkyl- oder Aralkyl- bzw. Alkylarylresten stehen beispielsweise für die den vorangehenden Alkylresten entsprechenden Alkylenreste.

Aryl steht für einen carbocyclischen aromatischen Rest mit 6 bis 34 Gerüstkohlenstoffatomen. Gleiches gilt für den aromatischen Teil eines Arylalkylrestes, auch Aralkylrest genannt, sowie für Arylbestandteile komplexerer Gruppen, wie z.B. Arylcarbonylresten.

**Arylalkyl** bzw. **Aralkyl** bedeutet jeweils unabhängig einen geradkettigen, verzweigten oder unverzweigten Alkyl-Rest nach vorstehender Definition, der einfach, mehrfach oder vollständig durch Aryl-Reste gemäß vorstehender Definition substituiert sein kann.

Die vorangehenden Aufzählungen sind beispielhaft und nicht als Limitierung zu verstehen.

Für die Herstellung der zu reinigenden Diarylcarbonate und als Verunreinigungen aus diesen zu entfernende Umesterungskatalysatoren können aus der Literatur bekannte Umesterungskatalysatoren für die Dialkylcarbonat-Phenol-Umesterung sein, wie z. B. Hydride, Oxide, Hydroxide, Alkoholate, Amide und andere Salze von Alkali- und Erdalkalimetallen, wie von Lithium, Natrium, Kalium, Rubidium, Caesium, Magnesium und Calcium, bevorzugt Lithium, Natrium, Kalium, Magnesium und Calcium und besonders bevorzugt Lithium, Natrium und Kalium (vgl. z.B. US 3 642 858, US 3 803 201 oder EP-A 1082). Salze der Alkali- und Erdalkalimetalle können auch solche von organischen oder anorganischen Säuren sein, wie von Essigsäure, Propionsäure, Buttersäure, Benzoesäure, Stearinsäure, Kohlensäure (Carbonate oder Hydrogencarbonate), Phosphorsäure, Blausäure, Rhodanwasserstoffsäure, Borsäure, Zinnsäure, C₁₄-Stannonsäuren oder Antimonsäure. Bevorzugt kommen als Verbindungen der Alkali- und Erdalkalimetalle die Oxide, Hydroxide, Alkoholate, Acetate, Propionate, Benzoate, Carbonate und Hydrogencarbonate in Frage, in besonders bevorzugter Weise werden Hydroxide, Alkoholate, Acetate, Benzoate oder Carbonate eingesetzt. Die genannten Alkali- oder Erdalkalimetallverbindungen werden bevorzugt in Mengen von 0,001 bis 2 Gew.-%, bevorzugt von 0,005 bis 0,9 Gew.-% und besonders bevorzugt von 0,01 bis 0,5 Gew.-%, bezogen auf das Gewicht des umzusetzenden Reaktionsgemischs, eingesetzt.

Weitere erfindungsgemäß einsetzbare Katalysatoren sind Metallverbindungen wie AlX₃, TiX₃, UX₄, TiX₄, VOX₃, VX₅, ZnX₂, FeX₃, PbX₂ und SnX₄, worin X für Halogen-, Acetoxy-, Alkoxy-oder Aryloxyreste steht (DE-OS 2 58 412). Besonders bevorzugte erfindungsgemäß einsetzbare Katalysatoren sind Metallverbindungen wie AlX₃, TiX₄, PbX₂ und SnX₄, wie beispielsweise Titantetrachlorid, Titantetramethoxid Titantetraphenoxid, Titantetraethoxid, Titantetraisopropylat, Titantetradodecylat, Zinntetraisooctylat und Aluminiumtriisopropylat. Ganz besonders bevorzugt sind Metallverbindungen TiX₄. Die genannten Metallverbindungen werden bevorzugt in Mengen von 0,001 bis 5 Gew.-%, bevorzugt von 0,005 bis 5 Gew.-% und besonders bevorzugt von 0,01 bis 5 Gew.-%, bezogen auf das Gewicht des umzusetzenden Reaktionsgemischs, eingesetzt.

Halogen bedeutet im Rahmen der Erfindung Fluor, Chlor oder Brom, bevorzugt Fluor oder Chlor, besonders bevorzugt Chlor.

Weitere erfindungsgemäß einsetzbare Katalysatoren sind zinnorganische Verbindungen der allgemeinen Formel (R¹¹)_{4-X}-Sn(Y)_{X}, in der Y für einen Rest OCOR¹², OH oder OR steht, wobei R¹² C₁-C₁₂-Alkyl, C₆-C₁₂-Aryl oder C₇-C₁₃-Alkylaryl bedeutet, R¹¹ unabhängig von R¹² die Bedeutung von R¹² hat und x eine ganze Zahl von 1 bis 3 bedeutet, Dialkylzinnverbindungen mit 1 bis 12 C-Atomen im Alkylrest oder Bis-(trialkylzinn)verbindungen, beispielsweise Trimethylzinnacetat, Triethylzinnbenzoat, Tributylzinnacetat, Triphenylzinnacetat, Dibutylzinndiacetat, Dibutylzinndilaurat, Dioctylzinndilaurat, Dibutylzinnadipinat, Dibutyldimethoxyzinn, Dimethylzinnglykolat, Dibutyldiethoxyzinn, Triethylzinnhydroxid, Hexaethylstannoxan, Hexabutylstannoxan, Dibutylzinnoxid, Dioctylzinnoxid, Butylzinntriisooctylat, Octylzinntriisooctylat, Butylstannonsäure und Octylstannonsäure in Mengen von 0,001 bis 20 Gew: % (vgl. EP 879, EP 880, EP 39 452, DE-OS 34 45 555, JP 79/63023), polymere Zinnverbindungen der Formel -[-RR¹¹Sn-O-]-, in welcher R und R¹¹ unabhängig voneinander die vorangehend für R¹² genannte Bedeutung haben, beispielsweise Poly[oxy(dibutylstannylen)] Poly[oxy(dioctylstannylen)], Poly[oxy(butylphenylstannylen)] und Poly[oxy(diphenylstannylen)] (DE-OS 34 45 552), polymere Hydroxystannoxane der Formel -[-RSn(OH)-O-]-, beispielsweise Poly(ethylhydroxystannoxan), Poly(butylhydroxystannoxan), Poly-(octylhydroxystannoxan), Poly(undecylhydroxystannoxan) und Poly(dodecylhydroxystannoxane) in Mengen von 0,001 bis 20 Gew.-%, bevorzugt von 0,005 bis 5 Gew.-%, bezogen auf Dialkylcarbonat (DE-OS 40 06 520). Weitere erfindungsgemäß einsetzbare Zinnverbindungen sind Sn(II)oxide der allgemeinen Formel

X-R₂Sn-O-R₂Sn-Y,

worin X und Y unabhängig voneinander OH, SCN, OR¹³, OCOR¹³ oder Halogen und R Alkyl, Aryl bedeuten soll, worin R¹³ die vorangehend für R¹² genannte Bedeutung hat (EP 0 338 760).

Als weitere erfindungsgemäß einsetzbare Katalysatoren kommen Bleiverbindungen, gegebenenfalls zusammen mit Triorganophosphanen, einer Chelatverbindung oder einem Alkalimetallhalogenid, beispielsweise Pb(OH)₂-2PbCO₃, Pb(OCO-CH₃)₂, Pb(OCO-CH₃)₂ ·2LiCl, Pb(OCO-CH₃)₂ • 2PPh₃ in Mengen von 0,001 bis 1, bevorzugt von 0,005 bis 0,25 Mol pro Mol Dialkylcarbonat (JP 57/176932, JP 01/093580), sowie andere Blei(II)- und Blei(IV)-verbindungen, wie PbO, PbO₂, Mennige, Plumbite, und Plumbate (JP 01/093560), Eisen(III)acetat (JP 61/1 72 852), weiterhin Kupfersalze und/oder Metallkomplexe, beispielsweise von Alkali, Zink, Titan und Eisen (JP 89/005588).

Bevorzugte Katalysatoren für das erfindungsgemäße Verfahren sind die vorangehend genannten Metallverbindungen AlX₃, TiX₃, UX₄, TiX₄, VOX₃, VX₅, ZnX₂, FeX₃, PbX₂ und SnX₄. Besonders bevorzugt sind AlX₃, TiX₄, PbX₂ und SnX₄, wovon beispielhaft Titantetrachlorid, Titantetramethoxid Titantetraphenoxid, Titantetraethoxid, Titantetraisopropylat, Titantetradodecylat, Zinntetraisooctylat und Aluminiumtriisopropylat genannt seien. Ganz besonders bevorzugt sind Metallverbindungen TiX₄. Insbesondere bevorzugt sind Titantetramethoxid, Titantetraphenoxid und Titantetraethoxid.

In bevorzugten Ausführungsformen enthält das zu reinigende Diarylcarbonat Verbindungen mit einem Siedepunkt zwischen dem des Diarylcarbonats und dem des während der Herstellung des Diarylcarbonats als Zwischenprodukt gebildeten Alkylarylcarbonats als Verunreinigung, welche in einem weiteren Seitenstrom der ersten Destillationskolonne entnommen werden. Diese weitere Seitenstromentnahme erfolgt in der ersten Destillationskolonne bevorzugt oberhalb der Seitenstromentnahme für das Diarylcarbonat.

Während der Herstellung des Diarylcarbonats als Zwischenprodukt gebildete Alkylarylcarbonate im Rahmen der Erfindung sind bevorzugt solche der allgemeinen Formel (IV) 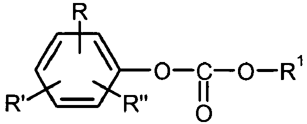 worin R, R' und R" die für die allgemeine Formel (I) und R¹ die für die allgemeine Formel (II) genannte Bedeutung haben können.

Bevorzugte Alkylarylcarbonate sind Methyl-phenyl-carbonat, Ethyl-phenyl-carbonat, Propyl-phenyl-carbonat, Butylphenyl-carbonat und Hexyl-phenyl-carbonat, Methyl-(o-kresyl)-carbonat, Methyl-(p-kresyl)-carbonat, Ethyl-(o-kresyl)-carbonat, Ethyl-(p-kresyl)-carbonat, Methyl- oder Ethyl-(p-chlorphenyl)-carbonat. Besonders bevorzugte Alkylarylcarbonate sind Methyl-phenyl-carbonat und Ethyl-phenyl-carbonat. Ganz besonders bevorzugt ist Methyl-phenyl-carbonat.

Bevorzugt enthält das aufzureinigende Diarylcarbonat - auch als Rohdiarylcarbonat bezeichnet-10 bis 90 Gew.-%, besonders bevorzugt 20 bis 80 Gew.-% und ganz besonders bevorzugt 40 bis 80 Gew.-% Diarylcarbonat sowie 5 bis 90 Gew.-%, besonders bevorzugt 5 bis 60 Gew.-% und ganz besonders bevorzugt 5 bis 40 Gew.-% Alkylarylcarbonat, 1 bis 90 Gew.-%, besonders bevorzugt 1 bis 50 Gew.-% und ganz besonders bevorzugt 1 bis 30 Gew.-% aromatische Hydroxyverbindung, 0 bis 5 Gew.-%, besonders bevorzugt 0 bis 2 Gew.-% und ganz besonders bevorzugt 0 bis 0,5 Gew.-% hochsiedende Nebenkomponenten, 0 bis 5 Gew.-%, besonders bevorzugt 0,0001 bis 2 Gew.-% und ganz besonders bevorzugt 0,0001 bis 1 Gew.-% mittelsiedende Nebenkomponenten und 0,01 bis 10 Gew.-%, besonders bevorzugt 0,1 bis 5 Gew.-% und ganz besonders bevorzugt 1 bis 5 Gew.-% Katalysator, wobei die Summe aller vorangehend genannten Komponenten im zu reinigenden Diarylcarbonat 100 Gew.-% ergibt. Die Gew.-%-Angaben sind jeweils bezogen auf das gesamte Gewicht des zu reinigenden Rohdiarylcarbonats.

Hochsiedende Nebenkomponenten - oft auch als Schwersieder bezeichnet - sind im Rahmen der Erfindung solche, deren Siedepunkt oberhalb desjenigen des Diarylcarbonats liegen. Mittelsiedende Komponenten - oft auch als Zwischensieder bezeichnet - sind im Rahmen der Erfindung solche, deren Siedepunkt zwischen dem des Diarylcarbonats und dem des während der Herstellung des Diarylcarbonats als Zwischenprodukt gebildeten Alkylarylcarbonats liegt.

Mit dem erfindungsgemäßen Verfahren können vorzugsweise Diarylcarbonate mit einer Reinheit von, d.h. einem Gehalt von reinem Diarylcarbonat von, 99 bis 100 Gew.-%, besonders bevorzugt 99,5 bis 100 Gew.-% und ganz besonders bevorzugt 99,9 bis 100 Gew.-%, bezogen auf das Gesamtgewicht des aufgereinigten Diarylcarbonats erhalten werden.

Das im Seitenstrom der ersten Destillationskolonne entnommene Diarylcarbonat kann flüssig oder dampfförmig der ersten Destillationskolonne entnommen werden. Bevorzugt wird das im Seitenstrom der ersten Destillationkolonne entnommene Diarylcarbonat dampfförmig der ersten Destillationskolonne entnommen. In bevorzugten Ausführungsformen kann jedoch die flüssige Entnahme des Diarylcarbonats im Seitenstrom insbesondere aufgrund konstruktiver Gegebenheiten bevorzugt sein.

Die erste Destillationskolonne weist mindestens zwei Sektionen, d.h. einen Verstärkungsteil im oberen Teil der Kolonne und einen Abtriebsteil im unteren Teil der Kolonne auf. Bevorzugt kann der Verstärkungsteil der ersten Destillationskolonne in einen unteren und einen oberen Verstärkungsteil unterteilt sein. Weiterhin bevorzugt kann der Abtriebsteil der ersten Destillationskolonne in einen unteren und einen oberen Abtriebsteil unterteilt sein.

Ingesamt weist die erste Destillationskolonne bevorzugt eine Gesamttrennleistung von 3 bis 160, besonders bevorzugt von 10 bis 90, ganz besonders bevorzugt von 13 bis 50 theoretische Stufen auf. Der obere Verstärkungsteil weist dabei bevorzugt eine Trennleistung von 0 bis 40, besonders bevorzugt 1 bis 20, ganz besonders bevorzugt 1 bis 10 theoretische Stufen, der untere Verstärkungsteil von bevorzugt 1 bis 40, besonders bevorzugt 5 bis 20, ganz besonders bevorzugt 5 bis 15 theoretische Stufen, der obere Abtriebsteil von bevorzugt 1 bis 40, besonders bevorzugt 2 bis 30, ganz besonders bevorzugt 5 bis 20 theoretische Stufen und der untere Abtriebsteil von bevorzugt 1 bis 40, besonders bevorzugt 2 bis 20, ganz besonders bevorzugt 2 bis 15 theoretische Stufen auf.

Die Verdampfung erfolgt vorzugsweise in einem Temperaturbereich von 100 bis 300°C, bevorzugt von 150 bis 240°C und besonders bevorzugt von 180 bis 230°C im Sumpf der Kolonne. Die Kondensation der Dämpfe am Kopf der Kolonne kann ein oder mehrstufig, bevorzugt ein- oder zweistufig, in einem Temperaturbereich von vorzugsweise 40 bis 250°C, bevorzugt 50 bis 200°C und besonders bevorzugt 60 bis 180°C erfolgen.

Das Sumpfprodukt der ersten Destillationskolonne hat einen Restgehalt an Diarylcarbonat von unter 95 Gew.-% bevorzugt unter 90 Gew.-% und besonders bevorzugt unter 75 Gew.-%. Zur Vermeidung von Katalysatorverlusten kann das Sumpfprodukt der ersten Destillationskolonne bevorzugt zu mindestens 50 %, besonders bevorzugt zu mindestens 80 % und ganz besonders bevorzugt zu mindestens 90 % wieder in die Umesterung aus wenigstens einem Dialkylcarbonat und wenigstens einer aromatischen Hydroxyverbindung zurückführt werden. Der restliche Teil des Sumpfproduktes der ersten Destillationskolonne kann einer Aufarbeitungsstufe, im Folgenden als Rückstandsaufkonzentrierung bezeichnet, zwecks Aufkonzentrierung des Rückstands und teilweisen Rückgewinnung des im Sumpfprodukt der ersten Destillationskolonne noch enthaltenen Diarylcarbonats zugeführt werden. Das in der Rückstandsaufkonzentrierung zurückgewonnene Diarylcarbonat kann in einer besonderen Ausführungsform des Verfahrens flüssig oder dampfförmig, bevorzugt dampfförmig, wieder der ersten Destillationskolonne zugeführt. Der aufkonzentrierte Rückstand aus der Rückstandsaufkonzentrierung kann entweder aus dem Verfahren ausgeschleust oder einer weiteren Aufarbeitungsstufe zwecks Rückgewinnung des Katalysators zugeführt werden. Dadurch können sowohl Verluste teurer Katalysatoren als auch Verluste an gewünschten Diarylcarbonat vermieden und damit dass erfindungsgemäße Verfahren zusätzlich wirtschaftlicher gestaltet werden.

Die erste Destillationskolonne wird bevorzugt bei einem Kopfdruck von 1 bis 1000 mbar (absolut), besonders bevorzugt von 1 bis 100 mbar (absolut) und ganz besonders bevorzugt von 5 bis 50 mbar (absolut) betrieben.

In bevorzugten Ausführungsformen des erfindungsgemäßen Verfahrens können Leitungen und Aggregate, die Gemische führen, welche einen Festpunkt von mehr als 30°C, bevorzugt mehr als 40°C aufweisen, auf Temperaturen oberhalb dieses Festpunktes, bevorzugt auf Temperaturen von mehr als 1°C oberhalb dieses Festpunktes, besonders bevorzugt auf Temperaturen von mehr als 5°C oberhalb dieses Festpunktes beheizt werden. Dadurch werden Ausfällungen von Feststoffen innerhalb dieser Leitungen und Aggregate vermieden und die Wiederinbetriebnahme der entsprechenden Anlagen nach Stillständen erheblich erleichtert.

Das erfindungsgemäße Verfahren wird beispielhaft anhand der Figuren 1 bis 5b beschrieben.
Fig. 1.1 beschreibt die Reinigung von Diarylcarbonat in einer Destillationskolonne.
Fig. 1.2 beschreibt eine nicht erfindungsgemäße Ausführung der Reinigung von Diarylcarbonat in einer Destillationskolonne mit einer zusätzlichen Rückstandsaufkonzentrierung.
Fig. 1.3 beschreibt eine nicht erfindungsgemäße Ausführung der Reinigung von Diarylcarbonat in einer Destillationskolonne mit einer zusätzlichen Rückstandsaufkonzentrierung in Form eines Verdampfers.
Fig. 2.1 beschreibt die Reinigung von Diarylcarbonat in einer Destillationskolonne mit zusätzlicher externer Seitenstromkolonne, welche lediglich als Verstärkungsteil ausgeführt ist.
Fig. 2.2 beschreibt die Reinigung von Diarylcarbonat in einer Destillationskolonne mit zusätzlicher integrierter Seitenstromkolonne, welche lediglich als Verstärkungsteil ausgeführt ist.
Fig. 2.3 beschreibt die Reinigung von Diarylcarbonat in einer Destillationskolonne mit zusätzlicher externer Seitenstromkolonne, welche einen Verstärkungsteil und einen Abtriebsteil aufweist.
Fig. 3.1 beschreibt die erfindungsgemäße Reinigung von Diarylcarbonat in einer Trennwandkolonne mit flüssiger Seitenstromentnahme des gereinigten Diarylcarbonats.
Fig. 3.2 beschreibt die Reinigung von Diarylcarbonat in einer Trennwandkolonne mit dampfförmiger Seitenstromentnahme des gereinigten Diarylcarbonats.
Fig. 4 beschreibt ausschnittweise die Kondensation am Kopf einer Destillations- oder Trennwandkolonne in einer oder mehreren zusätzlichen Kolonnensektion(en) mit einem in einem externen Kreislauf gekühlten Kondensat.
Fig. 5a beschreibt ausschnittweise die Kondensation am Kopf einer Destillations- oder Trennwandkolonne, wobei der Kolonnendurchmesser im Bereich der Kondensation unverändert bleibt.
Fig. 5b beschreibt ausschnittweise die Kondensation am Kopf einer Destillations- oder Trennwandkolonne mit einer Reduktion des Kolonnendurchmessers in Richtung Kolonnenkopf.

Die Figuren dienen der beispielhaften Erläuterung der Erfindung und sind nicht als Beschränkung aufzufassen.

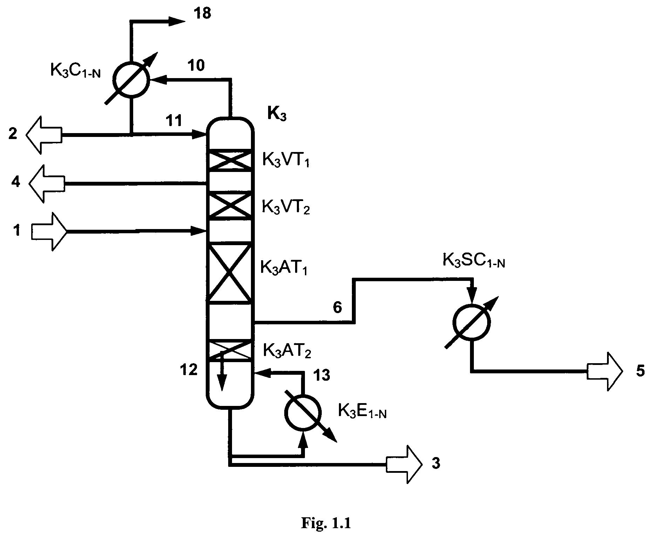

In den Fig. 1.1 bis 5b bedeuten
- K₃: erste Diarylcarbonat-Destillationskolonne mit Seitenstromentnahme
- K₃C_{1-N}: ein- oder mehrstufiger Kopfkondensator (Reihen und/oder Parallelschaltung)
- K₃E_{1-N}: ein- oder mehrstufiger Verdampfer für Sumpfprodukt (Reihen und/oder Parallelschaltung)
- K₃VT₁: oberer Verstärkungsteil
- K₃VT₂: unterer Verstärkungsteil
- K₃AT₁: oberer Abtriebsteil
- K₃AT₂: (unterer) Abtriebsteil
- K₃SC_{1-N}: ein- oder mehrstufiger Seitenstromkondensator (Reihen und/oder Parallelschaltung)
- K₄: Seitenstromkolonne
- K₄C_{1-N}: ein- oder mehrstufiger Kopfkondensator der Seitenstromkolonne (Reihen und/oder Parallelschaltung)
- K₄VT₁: Verstärkungsteil der Seitenstromkolonne
- K₄AT₁: Abtriebssteil der Seitenstromkolonne
- K₄E_{1-N}: ein- oder mehrstufiger Verdampfer für Sumpfprodukt der Seitenstromkolonne (Reihen und/oder Parallelschaltung)
- T: Trennwand
- K₃TLO: Verstärkungsteil auf der Zulaufseite der Trennwand
- K₃TLU: Abtriebsteil auf der Zulaufseite der Trennwand
- K₃TRU: Verstärkungsteil auf der Entnahmeseite der Trennwand
- K₃TRO: Abtriebsteil auf der Entnahmeseite der Trennwand
- K₃CS_{1-N}: ein oder mehrere Kololonnenabschnitt(e) zur Kondensation in der Destillations- oder Trennwandkolonne (Reihen und/oder Parallelschaltung)
- K₃W _{1-N}: ein oder mehrere Flüssigkeitskühler (Reihen und/oder Parallelschaltung)
- K₃RA: Rückstandsaufkonzentrierung
- K₃E_{N+1}: Rückstandsaufkonzentrierung in Form eines Verdampfers

Weiterhin sind in den Fig. 1.1 bis 5b die folgenden Stoffströme benannt
- 1: Rohdiarylcarbonat
- 2: Destillat der Kolonne K₃
- 3: Sumpfprodukt der Kolonne K₃
- 4: Zusätzlicher Seitenstrom der K₃ mit Zwischensiedern
- 5: Aufgereinigtes flüssiges Diarylcarbonat
- 6: Dampfförmiger Seitenstrom der K₃
- 7: Dämpfe am Kopf der Seitenstromkolonne K₄
- 8: Rücklauf der Seitenstromkolonne K₄
- 9: Sumpfprodukte der Kolonne K₄
- 10: Dämpfe aus K₃ zur Kondensation in K₃C_{1-N}
- 11: Rücklauf der Kolonne K₃
- 12: Flüssigkeit aus unterem Abtriebsteil der K₃
- 13: Dampf-/Flüssigkeitsgemisch aus K₃E_{1-N}
- 14: Flüssigkeit aus Verstärkungsteil nach K₃TLO (besondere Ausführung K₃ als Trennwandkolonne)
- 15: Flüssigkeit aus Verstärkungsteil nach K₃TRO (besondere Ausführung K₃ als Trennwandkolonne)
- 16: Dampf aus Abtriebsteil nach K₃TLU (besondere Ausführung K₃ als Trennwandkolonne)
- 17: Dampf aus Abtriebsteil nach K₃TRU (besondere Ausführung K₃ als Trennwandkolonne)
- 18: Nicht kondensierte Dämpfe und/oder Inerte nach Kondensation K₃C_{1-N}
- 19: Nicht kondensierte Dämpfe und/oder Inerte nach Kondensation K₄C_{1-N}
- 20: Diarylcarbonat enthaltender Strom aus K₃RA
- 21: Teilstrom aus Kondensationssektion K₃CS_{1-N} zum Kühler K₃W_{1-N}
- 22: Gekühltes Kondensat vom K₃W_{1-N} zur Kondensationssektion K₃CS_{1-N}
- 23: Flüssigkeit aus Abtriebsteil der Seitenstromkolonne K₄
- 24: Katalysatorhaltiger Strom zur Umesterung (Teilstrom aus Sumpf K₃)
- 25: Teilstrom aus Sumpfprodukt der K₃ zur K₃RA
- 26: Strom aus K₃RA zur Ausschleusung oder Katalysatorrückgewinnung

In einer bevorzugten Ausführungsform des erfindungsgemäßen Verfahrens wird die Reinigung des Diarylcarbonats in einer Destillationskolonne durchgeführt, welche wenigstens drei Sektionen aufweist. Bei diesen wenigstens drei Sektionen handelt es sich um wenigstens einen Verstärkungsteil und wenigstens einem Abtriebsteil, wobei der Abtriebsteil in einen unteren und einen oberen Abtriebsteil aufgeteilt ist. Besonders bevorzugt weist die Destillationskolonne mit einem Verstärkungsteil und einem Abtriebsteil, wobei der Abtriebsteil in einen unteren und einen oberen Abtriebsteil aufgeteilt ist, vier Sektionen auf, wobei auch der Verstärkungsteil in einen unteren und oberen Verstärkungsteil aufgeteilt ist.

Eine nicht erfindungsgemäße Variante des erfindungsgemäßen Verfahrens ist beispielhaft in Fig. 1.1 dargestellt. Die Destillationskolonne K₃ dieser bevorzugten Ausführungsform weist vier Sektionen, einen unteren Abtriebsteil (K₃AT₂) und einen oberen Abtriebsteil (K₃AT₁) sowie einen unteren Verstärkungsteil (K₃VT₂) und einen oberen Verstärkungsteil (K₃VT₁) auf. Das Rohdiarylcarbonat (1) wird zwischen unterem Verstärkungsteil K₃VT₂ und oberem Abtriebsteil K₃AT₁ der Kolonne zugeführt.

Der obere Verstärkungsteil weist bevorzugt eine Trennleistung von 0 bis 40, besonders bevorzugt 1 bis 20, ganz besonders bevorzugt 1 bis 10 theoretische Stufen, der untere Verstärkungsteil von bevorzugt 1 bis 40, besonders bevorzugt 5 bis 20, ganz besonders bevorzugt 5 bis 15 theoretische Stufen, der obere Abtriebsteil von bevorzugt 1 bis 40, besonders bevorzugt 2 bis 30, ganz besonders bevorzugt 5 bis 20 theoretische Stufen und der untere Abtriebsteil von bevorzugt 1 bis 40, besonders bevorzugt 2 bis 20, ganz besonders bevorzugt 2 bis 15 theoretische Stufen auf.

Zur Erreichung dieser Trennleistung der Sektionen können Füllkörper oder geordnete Packungen zum Einsatz kommen. Die zu verwendenden Füllkörper bzw. geordneten Packungen sind die für Destillationen üblichen, wie sie beispielsweise in Ullmann's Encyclopädie der Technischen Chemie, 4. Aufl,, Bd. 2, S. 528 ff. beschrieben sind. Als Beispiele für Füllkörper seien Raschig- oder Pall- und Novaloxringe, Berl-, Intalex- oder Torussättel, Interpackkörper und als Beispiele für geordnete Packungen seien Blech und Gewebepackungen (wie z.B. BX-Packungen, Montz Pak, Mellapak, Melladur, Kerapak und CY-Packung) aus verschiedenen Materialien, wie Glas, Steinzeug, Porzellan, Edelstahl, Kunststoff genannt. Bevorzugt sind Füllkörper und geordnete Packungen, die eine große Oberfläche, eine gute Benetzung sowie ausreichende Verweilzeit der Flüssigphase aufweisen. Diese sind beispielsweise Pall- und Novolaxringe, Berlsättel, BX-Packungen, Montz Pak, Mellapak, Melladur, Kerapak und CY-Packungen. Alternativ eignen sich auch Kolonnenböden, wie beispielesweise Sieb-, Glocken-, Ventil-, Tunnelböden. Bevorzugt werden Füllkörperschüttungen und strukturierte Packungen, besonders bevorzugt strukturierte Packungen eingesetzt.

Eine Sektion im Rahmen der Erfindung zeichnet sich dadurch aus, dass sich unter- und/oder oberhalb dieser Sektion eine Zufuhr- und/oder Entnahmestelle befindet. Bei der Verwendung von Füllkörperschüttungen und/oder strukturierten Packungen kann eine Sektion in mehrere Abschnitte unterteilt sein, wenn die Sektion mehr als 4, bevorzugt mehr als 10 und besonders bevorzugt mehr als 15 theoretische Stufen aufweist.

Die Destillationskolonne weist zudem einen ein- oder mehrstufigen (N-Stufigen) Kopfkondensator K₃C_{1-N} sowie einen ein- oder mehrstufigen (N-Stufigen) Verdampfer K₃E_{1-N} für das Sumpfprodukt auf. Bei Kondensation oder Verdampfung in mehreren Apparaten (Kondensatoren bzw. Verdampfern) sind sowohl Parallel- und/oder Reihenschaltungen als auch Kombinationen aus Parallel- und Reihenschaltung möglich.

Die Kondensation der Dämpfe am Kopf der Destillationskolonne kann ein oder mehrstufig, bevorzugt ein- oder zweistufig, in einem Temperaturbereich von 40 bis 250°C, bevorzugt von 50 bis 200°C und besonders bevorzugt von 60 bis 180°C erfolgen.

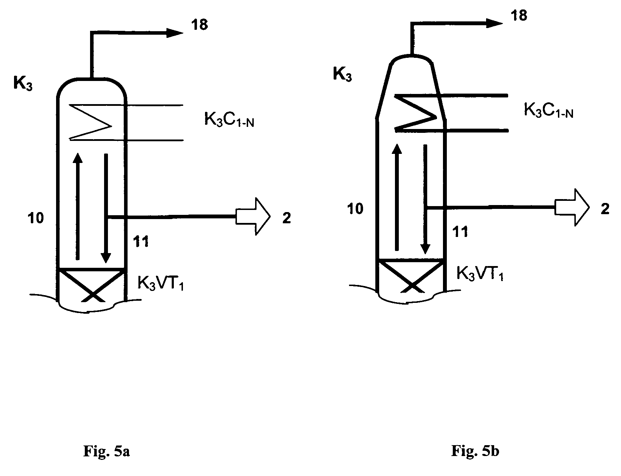

Hinsichtlich der Kondensation im Kopfkondensator sind unterschiedliche Ausführungsformen denkbar. Als Kopfkondensatoren eignen sich beispielsweise Rohrbündel- oder Plattenwärmetauscher. Das Verhältnis d₁/D₁ von Durchmesser der Dampfleitung von der Kolonne zum Kondensator (d₁) zu Kolonnendurchmesser der ersten Destillationskolonne (D₁) liegt bevorzugt im Bereich von 0,2 bis 1,0, besonders bevorzugt im Bereich von 0,5 bis 1. In einer besonders bevorzugten Ausführungsform kann der Kopfkondensator in die Destillationskolonne integriert sein, so dass zwischen Destillationskolonne und Kopfkondensator keine Dampfleitung erforderlich ist. Das Verhältnis d₁/D₁ beträgt in diesem Fall 1. Dabei kann der Kolonnenquerschnitt nach Eintritt in den Kopfkondensator unter Umständen auch dem Kondensationsfortschritt angepasst werden. Solche bevorzugten Ausführungsformen sind ausschnittsweise und beispielhaft in den Fig. 5a und 5b dargestellt.

Bei der in Fig. 5a dargestellten Ausführungsform bleibt der Kolonnendurchmesser im Bereich der Kondensation unverändert. Die aus dem Verstärkungsteil, falls vorhanden bevorzugt dem oberen Verstärkungsteil K₃VT₁, aufsteigenden Dämpfe (10) werden in dem oder den integrierten Kopfkondensator(en) K₃C_{1-N} kondensiert. Ein Teil des Kondensates wird als Rücklauf (11) wieder auf die obere Kolonnensektion aufgegeben. Der restliche Teil des Kondensats wird als Destillat (2) aus der Kolonne ausgeschleust. Inerte und/oder nicht kondensierte Dämpfe (18) werden am Kopf der Kolonne entnommen.

Bei einigen Kondensatorformen kann es von Vorteil sein, den Kolonnenquerschnitt variabel zu gestalten. Ist die Führung der zu kondensierenden Dämpfe beispielsweise von unten nach oben, so nimmt die Dampfmenge nach oben hin ab. Durch eine Reduktion des Kolonnendurchmessers in Richtung Kolonnenkopf, wird der für den Dampf verfügbare Kolonnenquerschnitt der nach oben hin abnehmenden Dampfmenge angepasst. Eine solche Ausführungsform ist beispielhaft in Fig. 5b dargestellt. Dabei muss die Entnahme der nicht kondensierten Dämpfe nicht zwangsläufig oben erfolgen. Wird beispielsweise eine Konstruktion gewählt, bei der ein Platten- oder Rohrbündel von oben in die Kolonne einfügt wird, so kann sich die Entnahme der nicht kondensierten Dämpfe auch seitlich befinden.

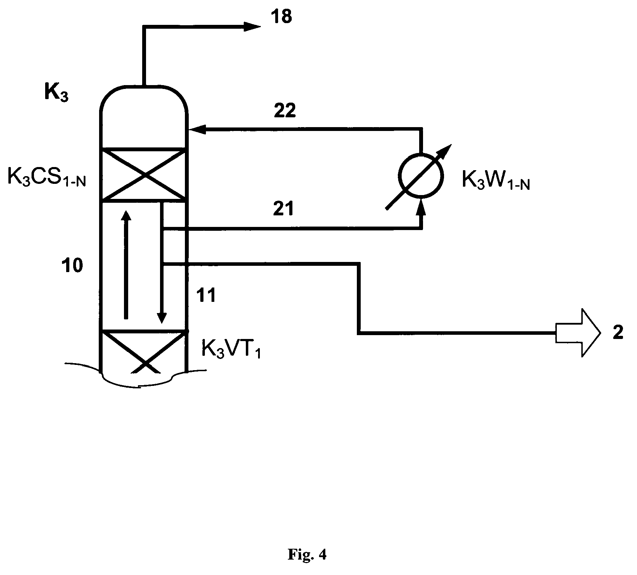

Eine weitere bevorzugte Ausführungsform eines Kopfkondensators wird in Fig. 4 dargestellt. Dabei erfolgt die Kondensation der aus dem Verstärkungsteil, falls vorhanden bevorzugt dem oberen Verstärkungsteil K₃VT₁, aufsteigenden Dämpfe (10) in einer oder mehreren zusätzlichen Kolonnensektion(en) (K₃CS_{1-N}) mit einem in einem externen Kreislauf gekühlten Kondensat. Die am unteren Ende dieser Kolonnensektion austretende Flüssigkeit wird dabei teilweise entnommen (21) und einem oder mehreren externen Kühler(n) K₃W_{1-N}, die sowohl in Reihe als auch parallel geschaltet sein können, zur Abfuhr der anfallenden Kondensationswärme zugeführt. Die restliche Flüssigkeit wird entweder als Destillat (2) ausgeschleust oder als Rücklauf (11) auf den Verstärkungsteil, falls vorhanden bevorzugt den oberen Verstärkungsteil K₃VT₁, aufgegeben. Nach der Kühlung wird die Flüssigkeit (22) oberhalb der zusätzlichen Kolonnensektion(en) K₃CS_{1-N} wieder der Destillationskolonne zugeführt. Bei der Kondensation in der Kolonne können dabei die bereits vorangehend für beschriebenen Kolonnenböden, Füllkörper oder strukturierten Packungen verwendet werden. Die Entnahme der nicht kondensierten Dämpfe oder Inerte (18) erfolgt oberhalb der Kolonnensektion(en) K₃CS_{1-N}.

Die aus dem unteren Abtriebsteil K₃AT₂ in der beispielhaften Darstellung in Fig. 1.1 ablaufende Flüssigkeit 12 wird in einer ein- oder mehrstufigen (N-stufigen) Verdampfung eingedampft, wobei die Dämpfe des dabei anfallenden Dampf-/Flüssigkeitsgemisches (13) wieder dem unteren Abtriebsteil K₃AT₂ zugeführt werden. Die Verdampfung erfolgt vorzugsweise in einem Temperaturbereich von 150 bis 300°C, bevorzugt von 160 bis 240°C und besonders bevorzugt von 180 bis 230°C im Sumpf der Kolonne. Die Temperatur am Kopf der Kolonne beträgt vorzugsweise 40 bis 250°C, bevorzugt 50 bis 200°C und besonders bevorzugt 60 bis 180°C. Man erhält ein Sumpfprodukt (3) mit einem Restgehalt von Diarylcarbonat von unter 95 Gew.-%, bevorzugt unter 90 Gew.-% und besonders bevorzugt unter 75 Gew.-%.

Beispielhaft wird das gereinigte Diarylcarbonat als dampfförmiger Seitenstrom (6) oberhalb des unteren Abtriebsteils K₃AT₂ entnommen und anschließend in einem ein- oder mehrstufigen (N-stufigen) Kondensator K₃SC_{1-N} kondensiert und als Flüssigkeit 5 abgeführt.

Die bei der Kondensation in dem oder den Kondensator(en) K₃SC_{1-N} anfallende Kondensationswärme kann bevorzugt zur Erzeugung von Heizdampf oder zur Beheizung anderer Verfahrensabschnitte, wie z.B. solchen bei der Herstellung von Diarylcarbonaten, verwendet werden.

Die Destillationskolonne K₃ wird bevorzugt bei einem Kopfdruck von 1 bis 1000 mbar (absolut), besonders bevorzugt von 1 bis 100 mbar (absolut) und ganz besonders bevorzugt von 5 bis 50 mbar (absolut) betrieben. Das Rücklaufverhältnis wird dabei so eingestellt, dass der Diarylcarbonat-Gehalt im Destillat 10 bevorzugt kleiner als 10 Gew.-%, besonders bevorzugt kleiner als 5 Gew.-% und ganz besonders bevorzugt kleiner 1 Gew.-%, bezogen auf das Gesamtgewicht des Destillats, beträgt. Hierzu wird bevorzugt ein Rücklaufverhältnis von 0,2 bis 5, besonders bevorzugt 0,2 bis 2 und ganz besonders bevorzugt von 0,3 bis 1,6 eingestellt, wobei das Rücklaufverhältnis im Rahmen der Erfindung dem Gewichtsverhältnis von in die Kolonne zurückgeführtem Kondensat zu am Kopf der Kolonne entnommenem Dampf ohne zurückgeführtes Kondensat entspricht.

Enthält das zu Rohdiarylcarbonat Verbindungen mit einem Siedepunkt zwischen dem des Diarylcarbonats und dem des während der Herstellung des Diarylcarbonats als Nebenprodukt gebildeten Alkylarylcarbonats als Verunreinigung, können diese erfindungsgemäß in einem weiteren Seitenstrom 4 der ersten Destillationskolonne entnommen werden.

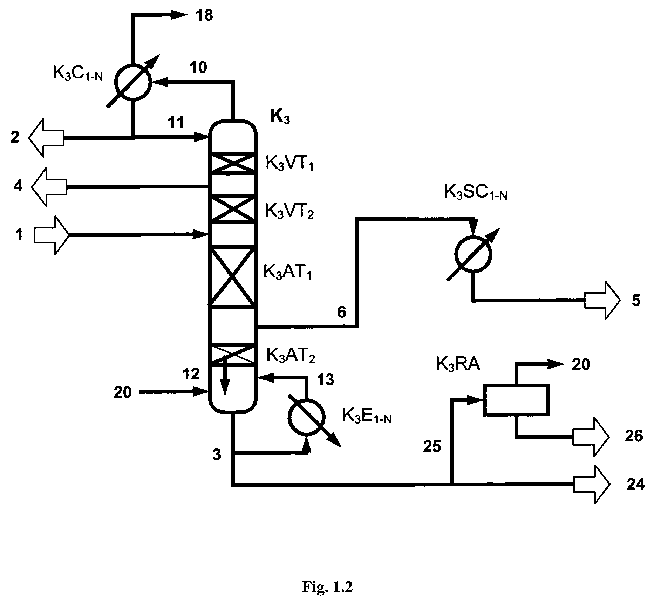

Wie in Fig. 1.2 dargestellt kann das Sumpfprodukt der ersten Destillationskolonne (3) zur Vermeidung von Katalysatorverlusten zu mindestens 50 %, bevorzugt mindestens 80 % und besonders bevorzugt mindestens 90 % wieder in die Umesterung aus wenigstens einem Dialkylcarbonat und wenigstens einer aromatischen Hydroxyverbindung zur Herstellung des Diraylcarbonats zurückgeführt werden (24). Der restliche Teil (25) des Sumpfproduktes kann einer Rückstandsaufkonzentrierung (K₃RA) zwecks Aufkonzentrierung des Rückstands und teilweiser Rückgewinnung des im Sumpfprodukt der ersten Destillationskolonne noch enthaltenen Diarylcarbonats zugeführt werden. Das in der Rückstandsaufkonzentrierung zurückgewonnene Diarylcarbonat (20) kann flüssig oder dampfförmig, bevorzugt dampfförmig wieder der ersten Destillationskolonne zugeführt werden. Der aufkonzentrierte Rückstand (26) kann entweder aus dem Verfahren ausgeschleust oder einer weiteren Aufarbeitungsstufe zwecks Rückgewinnung des Katalysators zugeführt werden.

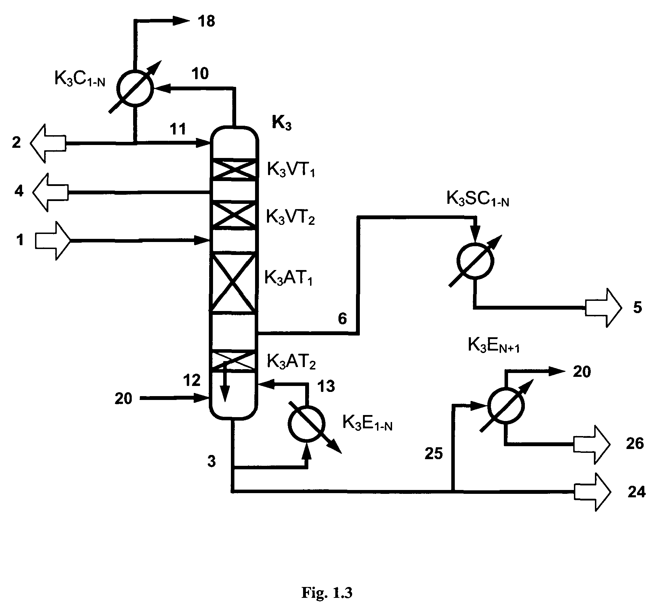

In einer Ausführungsform, die beispielhaft in Fig. 1.3 dargestellt ist, erfolgt die Aufkonzentrierung des Rückstands (25) mittels eines Verdampfers (K₃E_{N+1}). Die bei der Verdampfung anfallenden Dämpfe (20) werden in den Sumpf der ersten Destillationskolonne (K₃) geleitet. Auch dabei kann der aufkonzentrierte Rückstand (26) entweder aus dem Verfahren ausgeschleust oder einer weiteren Aufarbeitungsstufe zwecks Rückgewinnung des Katalysators zugeführt werden.

Im Falle einer Ausschleusung des aufkonzentzierten Rückstands (26) beträgt der Verlust an Diarylcarbonat weniger als 2 %, bevorzugt weniger als 1 %, besonders bevorzugt weniger als 0,5 % bezogen auf die Mengen an aufgereinigtem Diarylcarbonat..

Die vorangehend beschriebene Aufarbeitung des Sumpfproduktes der ersten Destillationskolonne kann optional auch bei allen weiteren im Folgenden vorgestellten Ausführungsformen durchgeführt werden.

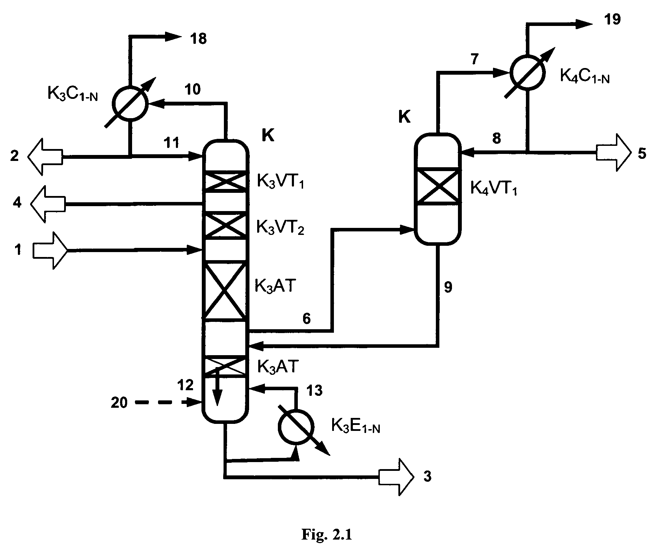

In einer besonders bevorzugten Ausführungsform des erfindungsgemäßen Verfahrens wird das im Seitenstrom der ersten Destillationskolonne entnommene Diarylcarbonat in wenigstens einer, bevorzugt in einer weiteren Destillationskolonne aufgereinigt. Eine solche weitere Destillationskolonne zur Aufreinigung des im Seitenstrom der ersten Destillationskolonne entnommenen Diarylcarbonats wird im Folgenden auch kurz als Seitenstromkolonne bezeichnet. In einer besonders bevorzugten Variante dieser bevorzugten Ausführungsform ist diese weitere Destillationskolonne (Seitenstromkolonne) ohne Abtriebsteil ausgeführt. Eine nicht erfindungsgemäße Ausführungsform ist beispielhaft in Fig. 2.1 dargestellt.

In dieser Variante wird das Diarylcarbonat in einer Destillationskolonne K₃ - wie sie bereits im Zusammenhang mit Fig. 1.1 beschrieben wurde - und einer zusätzlichen Seitenstromkolonne K₄ aufgereinigt. Der dampfförmige Seitenstrom 6 wird der Seitenstromkolonne K₄, bevorzugt deren unterem Teil zugeführt. Gegenüber der Ausführung in Fig. 1.1 weist die Destillationskolonne K₃ einen zusätzlichen Zulauf 9 oberhalb des unteren Abtriebsteils K₃AT₂ auf, über den das flüssige Sumpfprodukt aus der Seitenstromkolonne K₄ wieder in die K₃ zurückgeführt wird.

Die Seitenstromkolonne K₄ weist mindestens eine Sektion auf.

Insbesondere wird sie wie in Fig. 2.1 dargestellt als reiner Verstärkungsteil K₄VT₁ betrieben und besitzt vorzugsweise eine Trennleistung von 1 bis 50, besonders bevorzugt von 2 bis 30 und ganz besonders bevorzugt von 5 bis 20 theoretischen Stufen.

Die Seitenstromkolonne K₄ wird bei einem Kopfdruck von 1 bis 1000 mbar (absolut), besonders bevorzugt von 1 bis 100 mbar (absolut) und ganz besonders bevorzugt von 5 bis 50 mbar (absolut) betrieben. In der Seitenstromkolonne wird vorzugsweise ein Rücklaufverhältnis von 0,1 bis 10, besonders bevorzugt von 0,2 bis 5 und ganz besonders bevorzugt von 0,2 bis 2 eingestellt.

Die Kondensation der Dämpfe (7) am Kopf der Seitenstromkolonne K₄ kann ein- oder mehrstufig in einem Kopfkondensator K₄C_{1-N} erfolgen. Sie erfolgt bevorzugt ein oder zweistufig in einem Temperaturbereich von 70 bis 250°C, besonders bevorzugt von 90 bis 230°C und ganz besonders bevorzugt von 90 bis 210°C. Die bei der Kondensation anfallende Abwärme kann bevorzugt zur Erzeugung von Heizdampf oder zur Beheizung anderer Verfahrensabschnitte, wie z.B. solchen bei der Herstellung von Diarylcarbonaten, verwendet werden. Das bei der Kondensation anfallende Kondensat wird teilweise wieder als Rücklauf (8) auf die Seitenstromkolonne aufgegeben. Der restliche Teil des Kondensats wird als Destillat (5) (aufgereinigtes Diarylcarbonat) entnommen. Inerte und/oder nicht kondensierte Dämpfe (19) werden ausgeschleust.

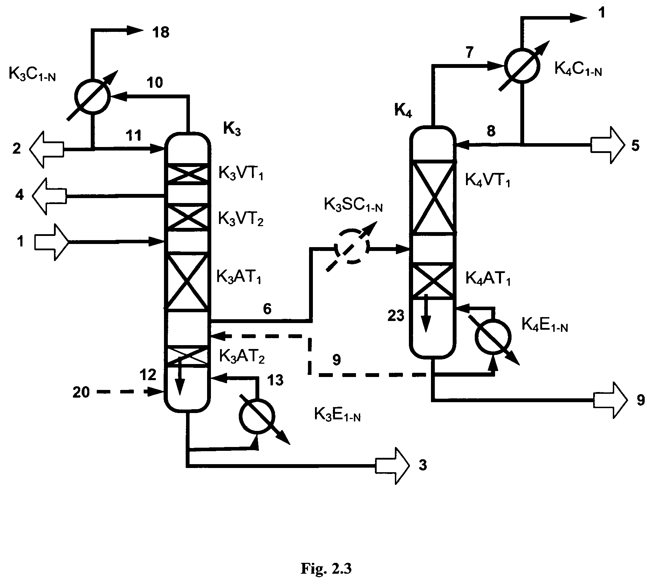

Hinsichtlich der Kondensation im Kondensator K₄C_{1-N} sind die gleichen Ausführungsformen, wie bereits für die Kondensation am Kopf der Destillationskolonne K₃ beschrieben (K₃C_{1-N}) geeignet. In einer weiteren besonders bevorzugten Variante der besonders bevorzugten Ausführungsform des erfindungsgemäßen Verfahrens unter Verwendung einer weiteren Destillationskolonne (Seitenstromkolonne) wird diese weitere Destillationskolonne (Seitenstromkolonne) sowohl mit wenigstens einem Verstärkungsteil als auch mit wenigstens einem Abtriebsteil ausgeführt. Eine nicht erfindungsgemäße Variante dieser Ausführungsform ist beispielhaft in Fig. 2.3 dargestellt.

Die in Fig. 2.3 dargestellte Seitenstromkolonne K₄ weist sowohl einen Abtriebsteil K₄AT₁ als auch einen Verstärkungsteil K₄AT₂ auf. Der dampfförmige Seitenstrom 6 der ersten Destillationskolonne K₃ kann dabei zunächst in einem ein- oder mehrstufigen Seitenstromkondensator K₃SC_{1-N} kondensiert und anschließend der Seitenstromkolonne K₄ zugeführt werden. Die Seitenstromkolonne K₄ wird bei einem Kopfdruck von 1 bis 1000 mbar (absolut), bevorzugt 1 bis 100 mbar (absolut) und besonders bevorzugt 5 bis 50 mbar (absolut) betrieben. Dabei ergibt sich im Sumpf eine Temperatur von 150 bis 300 °C, bevorzugt von 160 bis 240 °C und besonders bevorzugt 180 bis 230°C °C.

Die Seitenstromkolonne K₄ gemäß Fig. 2.3 hat eine Gesamttrennleistung von 5 bis 100 Stufen, bevorzugt 10 bis 80 Stufen besonders bevorzugt 30 bis 80 Stufen, wobei der Verstärkungsteil davon eine Trennleistung von 1 bis 99, bevorzugt von 1 bis 79 und besonders bevorzugt von 2 bis 79 aufweist. Die Seitenstromkolonne K₄ wird bei einem Rücklaufverhältnis von 0,5 bis 20, bevorzugt 1 bis 10 und besonders bevorzugt 1 bis 5 betrieben.

Die Kondensation der Dämpfe (7) am Kopf der Seitenstromkolonne K₄ kann ein- oder mehrstufig in einem Kopfkondensator K₄C_{1-N} erfolgen. Sie erfolgt bevorzugt ein oder zweistufig in einem Temperaturbereich von 70 bis 250°C, besonders bevorzugt von 90 bis 230°C und ganz besonders bevorzugt von 90 bis 210°C. Die bei der Kondensation anfallende Abwärme kann bevorzugt zur Erzeugung von Heizdampf oder zur Beheizung anderer Verfahrensabschnitte, wie z.B. solchen bei der Herstellung von Diarylcarbonaten, verwendet werden. Das bei der Kondensation anfallende Kondensat wird teilweise wieder als Rücklauf (8) auf die Seitenstromkolonne aufgegeben. Der restliche Teil des Kondensats wird als Destillat (5) (aufgereinigtes Diarylcarbonat) entnommen. Nicht kondensierte Dämpfe (19) werden ausgeschleust.

Die Verdampfung der aus dem Abtriebsteil K₄AT₁ der Seitenstromkolonne ablaufenden Flüssigkeit (23) kann ebenfalls ein- oder mehrstufig in einem Verdampfer K₄E_{1-N} erfolgen.

Das Sumpfprodukt (9) der Seitenstromkolonne K₄ kann anschließend ganz oder teilweise aus dem Verfahren ausgeschleust werden und/oder ganz oder teilweise wieder der Destillationskolonne K₃ zugeführt werden.

Die vorangehend beschriebene besonders bevorzugte Ausführungsform des erfindungsgemäßen Verfahrens unter Verwendung einer Seitenstromkolonne eignet sich insbesondere für die Reinigung von Diarylcarbonaten mit erhöhten Anforderungen bezüglich deren Qualität. Solche erhöhten Anforderungen können beispielsweise in einem reduzierten Anteil hochsiedender Nebenkomponenten liegen, wobei deren Anteil im Diarylcarbonat um 10 bis 100 Gew.-%, bevorzugt 20 bis 90 Gew.-% und besonders bevorzugt 25 bis 80 Gew.-% gegenüber dem Verfahren mit nur einer Destillationskolonne reduziert werden kann.

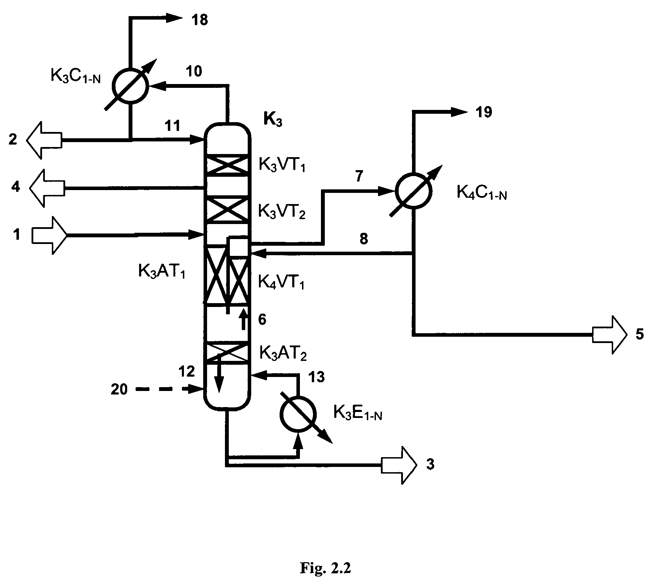

In einer besonders bevorzugten Ausführungsform des erfindungsgemäßen Verfahrens ist die weitere Destillationskolonne in die erste Destillationskolonne integriert. Für den Fall der besonders bevorzugten Variante, dass die weitere Destillationskolonne ohne Abtriebsteil ausgeführt ist, ist der Verstärkungsteil dieser weiteren Kolonne in die erste Destillationskolonne (K₃) integriert. Eine Variante dieser Ausführungsform ist beispielhaft in Fig. 2.2 dargestellt. Dabei gelangt ein Teil der aus dem unteren Abtriebsteil der ersten Destillationskolonne (K₃AT₂) kommenden Dämpfe (6) in einen integrierten Verstärkungsteil (K₄VT₁), um den Gehalt an Hochsiedern zu reduzieren. Die am Kopf dieser integrierten Seitenstromkolonne austretenden Dämpfe (7) werden in dem oder den externen Kondensator(en) K₄C_{1-N} kondensiert und teilweise als Rücklauf (8) wieder auf den Kopf der integrierten Seitenstromkolonne zurückgeführt. Der restliche Teil des Kondensats wird als Destillat (5) (gereinigtes Diarylcarbonat) entnommen. Nicht kondensierte Dämpfe (19) werden ausgeschleust.

Erfindungsgemäß ist die erste Destillationskolonne als Trennwandkolonne ausgeführt.

Trennwandkolonnen eignen sich ebenfalls zur Trennung eines Gemisches in drei Fraktionen, d.h. Kopfprodukt, Sumpfprodukt und Seitenstrom mit hoher Reinheit. Die Trennwandkolonne verfügt über eine in der Regel vertikal angeordnete Trennwand, welche die Zulaufseite von der Entnahmeseite für den Seitenstrom voneinander trennt. Die Trennwand ist dabei bevorzugt nicht über die gesamte Länge der Kolonne durchgängig. Meist befindet sich oberhalb der Trennwand noch ein Verstärkungsteil und unterhalb der Trennwand noch ein Abtriebsteil. Im Bereich der Trennwand befinden sich sowohl auf der Zulauf- als auch auf der Entnahmeseite für den Seitenstrom bevorzugt mindestens 2 Sektionen. Auf der Zulaufseite dient eine obere Sektion zur Reduktion der im Zulauf enthaltenen Schwersieder und eine untere Sektion zur Reduktion der im Zulauf enthaltenen Leichtsieder. Auf der Entnahmeseite für den Seitenstrom befindet sich ebenfalls eine obere Sektion oberhalb der Entnahme, welche zur Reduzierung der Leichtsieder aus dem Verstärkungsteil dient. Eine untere Sektion, die unterhalb der Entnahme angeordnet ist dient der Reduzierung von Schwersiedern, welche aus dem Abtriebsteil unterhalb der Trennwand kommen.

Die Aufteilung der aus dem Verstärkungsteil ablaufenden Flüssigkeit richtet sich nach den Anforderungen an die spezielle Trennaufgabe und kann durch konstruktive als auch regelungstechnische Maßnahmen beeinflusst werden. Gleiches gilt für den, aus dem Abtriebsteil kommenden, Dampfstrom.

Trennwandkolonnen sind dem Fachmann bekannt und beispielsweise in DE-A 33 02 525 oder DE-A 199 14 966 beschrieben.

Bevorzugt weist die Trennwandkolonne zusätzlich zu wenigstens einem Verstärkungsteil im oberen Teil der Kolonne und wenigstens einem Abtriebsteil im unteren Teil der Kolonne jeweils eine obere und untere Sektion auf der Zulaufseite der Trennwand und auf der Entnahmeseite der Trennwand auf, wobei Zulauf und Entnahme jeweils zwischen der oberen und unteren Sektion erfolgen.

In besonders bevorzugten Ausführungsformen des erfindungsgemäßen Verfahrens weist der Verstärkungsteil der Trennwandkolonne zwei Sektionen, d.h. einen unteren Verstärkungsteil und einen oberen Verstärkungsteil, auf.

In ganz besonders bevorzugten Ausführungsformen des erfindungsgemäßen Verfahrens weist die Trennwandkolonne mindestens sieben Sektionen auf, umfassend mindestens einem Abtriebsteil im unteren Teil der Kolonne, jeweils eine obere und untere Sektion auf der Zulaufseite der Trennwand und auf der Entnahmeseite der Trennwand sowie einen oberen und unteren Verstärkungsteil im oberen Teil der Kolonne auf.

Die Trennwand in einer solchen Trennwandkolonne ist bevorzugt in Längsrichtung der Kolonne angeordnet. Sie verhindert sowohl dampfseitigen als auch flüssigkeitsseitigen Stoffaustausch zwischen Zulauf- und Entnahmeseite.

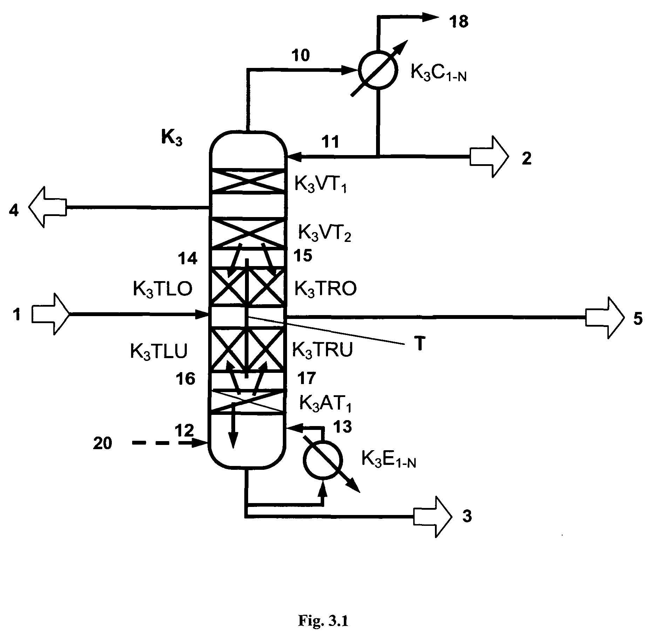

Eine beispielhafte Ausführungsform des erfindungsgemäßen Verfahrens mit einer Trennwandkolonne mit sieben Sektionen ist beispielhaft in Fig. 3.1 dargestellt. Die Trennwandkolonne K₃ in Fig. 3.1 weist einen Abtriebsteil K₃AT₁ im unteren Teil der Kolonne K₃, jeweils eine obere Sektion K₃TLO und untere Sektion K₃TLU auf der Zulaufseite der Trennwand T und eine obere Sektion K₃TRO und untere Sektion K₃TRU auf der Entnahmeseite der Trennwand T sowie einen oberen Verstärkungsteil K₃VT₁ und unteren Verstärkungsteil K₃VT₂ im oberen Teil der Kolonne auf. Das Rohdiarylcarbonat (1) wird zwischen der oberen Sektion K₃TLO und der unteren Sektion K₃TLU auf der Zulaufseite der Trennwand T der Kolonne zugeführt, das aufgereinigte Diarylcarbonat (5) wird zwischen der oberen Sektion K₃TRO und der unteren Sektion K₃TRU auf der Entnahmeseite der Trennwand T der Kolonne entnommen.

Die auf der Zulaufseite der Trennwand befindliche obere Sektion K₃TLO dient zur Abtrennung von Schwersiedern, die im Zulauf enthalten sind. Die untere Sektion K₃TLU auf der Zulaufseite der Trennwand dient zur Abtrennung von Leichtsiedem, die im Rohdiarylcarbonat (1) enthalten sind. Die auf der Entnahmeseite der Trennwand befindliche obere Sektion K₃TRO dient zur Abtrennung von Leichtsiedern, die im aus dem Verstärkungsteil K₃VT₂ im oberen Teil der Kolonne austretenden Flüssigkeitsstrom (15) enthalten sind. Die untere Sektion der Entnahmeseite K₃TRU dient zur Abtrennung von Schwersiedern, die im aus dem Abtriebsteil K₃AT₁ austretenden Dampfstrom (17) enthalten sind.

Die Entnahme des gereinigten Diarylcarbonats auf der Entnahmeseite der Trennwand kann flüssig oder dampfförmig erfolgen. Beim Kolonnendesign kann die Art der Entnahme die Anordnung der Trennwand innerhalb der Kolonne mitunter deutlich beeinflussen. Die Trennwand kann jeweils zur Entnahmeseite oder zur Zulaufseite verschoben in der Kolonne angeordnet sein und so den Querschnitt der jeweiligen Seite gegenüber der anderen verkleinern oder vergrößern. Im Falle dampfförmiger Entnahme des gereinigten Diarylcarbonats auf der Entnahmeseite der Trennwand ist der Querschnitt der Entnahmeseite der Kolonne bevorzugt größer als der Querschnitt der Zulaufseite, d.h. es gelangt mehr Dampf aus dem Abtriebsteil in die Entnahmeseite. Im Falle flüssiger Entnahme des gereinigten Diarylcarbonats auf der Entnahmeseite der Trennwand ist der Querschnitt der Zulaufseite der Kolonne bevorzugt identisch zum Querschnitt der Entnahmeseite.

Im Falle der flüssigen Entnahme des gereinigten Diarylcarbonats, die beispielhaft in Fig. 3.1 dargestellt ist, werden 10 bis 90 %, bevorzugt 20 bis 90 % besonders bevorzugt 50 bis 85 % der aus dem Abtriebsteil K₃TRO der Entnahmeseite ablaufenden Flüssigkeit als Seitenstrom 5 entnommen. Die restliche Flüssigkeit wird der unteren Sektion K₃TRU der Entnahmeseite zugeführt. Die aus dem Verstärkungsteil K₃VT₂ ablaufende Flüssigkeit wird zu 5 bis 50 %, bevorzugt zu 10 bis 50 % und besonders bevorzugt 10 bis 40 % auf die Zulaufseite der Trennwand, also oberhalb von K₃TLO, aufgegeben (14). Die restliche Flüssigkeit wird am oberen Ende der Entnahmeseite der Tennwand, also auf K₃TRO aufgegeben (15). Der aus dem Abtriebsteil K₃AT₁ aufsteigende Dampf wird zu 5 bis 90 %, bevorzugt zu 10 bis 80 und besonders bevorzugt zu 20 bis 75 % der Zulaufseite der Trennwand zugeführt (16).

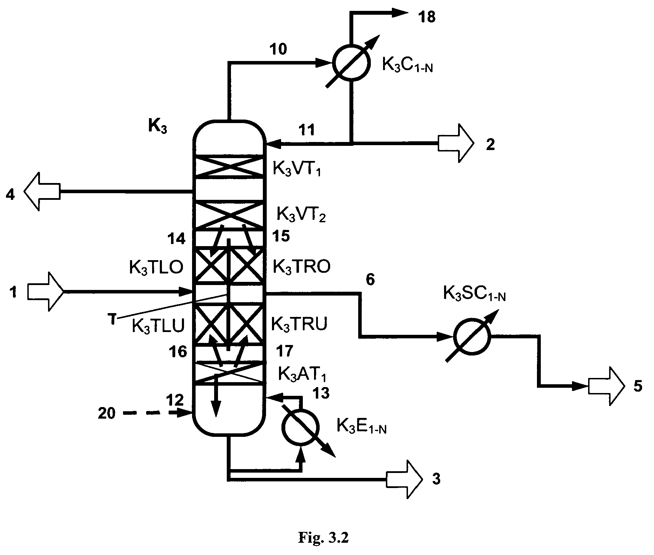

Im Falle der dampfförmigen Entnahme des gereinigten Diarylcarbonats, die beispielhaft in Fig. 3.2 dargestellt ist, werden 10 bis 90 %, bevorzugt 20 bis 90 % besonders bevorzugt 50 bis 85 % des aus der unteren Sektion K₃TRU der Entnahmeseite austretenden Dampfes als Seitenstrom (6) entnommen. Der restliche Dampf wird der oberen Sektion K₃TRO der Entnahmeseite zugeführt. Die aus dem Verstärkungsteil K₃VT₂ ablaufende Flüssigkeit wird zu 5 bis 90 %, bevorzugt zu 10 bis 80 % und besonders bevorzugt 20 bis 60 % auf die Zulaufseite der Trennwand, also oberhalb von K₃TLO, aufgegeben (14). Die restliche Flüssigkeit wird am oberen Ende der Entnahmeseite der Tennwand, also auf K₃TRO aufgegeben (15). Der aus dem Abtriebsteil K₃AT₁ aufsteigende Dampf wird zu 5 bis 90 %, bevorzugt zu 10 bis 80 und besonders bevorzugt zu 20 bis 60 % der Zulaufseite der Trennwand zugeführt (16). Im Falle der dampfförmigen Entnahme wird der dampfförmig entnommene Seitenstrom (6) bevorzugt in einem oder mehreren Seitenstromkondensator(en) K₃SC_{1-N} kondensiert und als flüssiger Diarylcarbonatstrom (5) abgeführt.

Der obere Verstärkungsteil einer solchen Trennwandkolonne weist bevorzugt eine Trennleistung von 0 bis 40, besonders bevorzugt 1 bis 20, ganz besonders bevorzugt 1 bis 10 theoretische Stufen, der untere Verstärkungsteil von bevorzugt 1 bis 40, besonders bevorzugt 5 bis 20, ganz besonders bevorzugt 5 bis 15 theoretische Stufen, der Abtriebsteil von bevorzugt 1 bis 40, besonders bevorzugt 2 bis 20, ganz besonders bevorzugt 2 bis 15 theoretische Stufen auf. Die obere Sektion K₃TLO und untere Sektion K₃TLU auf der Zulaufseite der Trennwand sowie die obere Sektion K₃TRO und untere Sektion K₃TRU auf der Entnahmeseite der Trennwand weisen bevorzugt jeweils eine Trennleistung von 1 bis 40, besonders bevorzugt 2 bis 20, ganz besonders bevorzugt 5 bis 20 theoretische Stufen auf.

Zur Erreichung dieser Trennleistung der Sektionen können Füllkörper oder geordnete Packungen zum Einsatz kommen. Die zu verwendenden Füllkörper bzw. geordneten Packungen sind die für Destillationen üblichen, wie sie beispielsweise in Ullmann's Encyclopädie der Technischen Chemie, 4. Aufl,, Bd. 2, S. 528 ff. beschrieben sind. Als Beispiele für Füllkörper seien Raschig- oder Pall- und Novaloxringe, Berl-, Intalex- oder Torussättel, Interpackkörper und als Beispiele für geordnete Packungen seien Blech und Gewebepackungen (wie z.B. BX-Packungen, Montz Pak, Mellapak, Melladur, Kerapak und CY-Packung) aus verschiedenen Materialien, wie Glas, Steinzeug, Porzellan, Edelstahl, Kunststoff genannt. Bevorzugt sind Füllkörper und geordnete Packungen, die eine große Oberfläche, eine gute Benetzung sowie ausreichende Verweilzeit der Flüssigphase aufweisen. Diese sind beispielsweise Pall- und Novolaxringe, Berlsättel, BX-Packungen, Montz Pak, Mellapak, Melladur, Kerapak und CY-Packungen. Alternativ eignen sich auch Kolonnenböden, wie beispielesweise Sieb-, Glocken-, Ventil-, Tunnelböden. Bevorzugt werden Füllkörperschüttungen und strukturierte Packungen, besonders bevorzugt strukturierte Packungen eingesetzt.

Die Trennwandkolonne weist zudem einen ein- oder mehrstufigen (N-Stufigen) Kopfkondensator K₃C_{1-N} sowie einen ein- oder mehrstufigen (N-Stufigen) Verdampfer K₃E_{1-N} für das Sumpfprodukt auf.

Die Kondensation der Dämpfe am Kopf der Trennwandkolonne kann ein oder mehrstufig, bevorzugt ein- oder zweistufig, in einem Temperaturbereich von 40 bis 250°C, bevorzugt von 50 bis 200°C und besonders bevorzugt von 60 bis 180°C erfolgen. Hinsichtlich der Kondensation im Kopfkondensator sind die vorangehend bereits für die Destillationskolonnen genannten unterschiedlichen Ausführungsformen der Kondensatoren möglich.

Die aus dem Abtriebsteil K₃AT₁ ablaufende Flüssigkeit (12) wird in einer ein- oder mehrstufigen (N-stufigen) Verdampfung eingedampft, wobei die Dämpfe des dabei anfallenden Dampf-/Flüssigkeitsgemisches (13) wieder dem unteren Abtriebsteil K₃AT₁ zugeführt werden. Die Verdampfung erfolgt vorzugsweise in einem Temperaturbereich von 100 bis 250°C, bevorzugt von 150 bis 240°C und besonders bevorzugt von 180 bis 220°C.

Die Trennwandkolonne wird bei einem Kopfdruck von 1 bis 1000 mbar (absolut), besonders bevorzugt von 1 bis 100 mbar (absolut) und ganz besonders bevorzugt von 5 bis 50 mbar (absolut) betrieben. Das Rücklaufverhältnis wird dabei so eingestellt, dass der Diarylcarbonat-Gehalt im Destillat 10 bevorzugt kleiner als 10 Gew.-%, besonders bevorzugt kleiner als 5 Gew.-% und ganz besonders bevorzugt kleiner 1 Gew.-%, bezogen auf das Gesamtgewicht des Destillats, beträgt. Hierzu wird bevorzugt ein Rücklaufverhältnis von 0,2 bis 5, besonders bevorzugt 0,2 bis 2 und ganz besonders bevorzugt von 0,3 bis 1,6 eingestellt, wobei das Rücklaufverhältnis im Rahmen der Erfindung dem Gewichtsverhältnis von in die Kolonne zurückgeführtem Kondensat zu am Kopf der Kolonne entnommenem Dampf ohne zurückgeführtes Kondensat entspricht.

Enthält das zu reinigende Diarylcarbonat Verbindungen mit einem Siedepunkt zwischen dem des Diarylcarbonats und dem des während der Herstellung des Diarylcarbonats als Zwischenprodukt gebildeten Alkylarylcarbonats als Verunreinigung, können diese erfindungsgemäß in einem weiteren Seitenstrom 4 der Trennwandkolonne entnommen werden.

Die folgenden Beispiele dienen der beispielhaften Erläuterung der Erfindung und sind nicht als Beschränkung aufzufassen.

### Beispiele

Im folgenden werden die folgenden Abkürzungen mit den entsprechenden Bedeutungen verwendet:
- DMC:: Dimethylcarbonat
- MPC:: Methylphenylcarbonat
- DPC:: Diphenylcarbonat
- Ti(PhO)₄:: Titantetraphenolat
- Salol:: Salicylsäurephenylester
- DPE:: Diphenylether

### Beispiel 1 (Vergleichsbeispiel): DPC-Aufreinigung in einer ersten Destillationskolonne (Feindestillation) mit Seitenstromkolonne gemäß Fig. 2.1

Aus einer zweistufigen Umesterung erhält man 224,4 kg/h eines DPC-haltigen Stroms mit 63,8/22,7/10,3/0,4/2,7/0,08 Gew.-% DPC/MPC/Phenol/DMC/Ti(PhO)₄/Salol. Dieser Strom wird in einer destillativen Aufarbeitung aus einer ersten Destillationskolonne (Feindestillation) mit dampfförmiger Seitenstromentnahme (K₃) und einer Seitenstromkolonne (K₄) gemäß Fig. 2.1 aufgearbeitet. Zusätzlich werden 1,3 kg/h eines dampfförmigen Gemisches aus einer Rückstandsaufkonzentierung mit 99,6 Gew.-% DPC am Sumpf der Kolonne K₃ zugeführt.

Die Feindestillation (K₃) besteht aus vier Sektionen, einem oberen Verstärkungsteil mit 2, einem unteren Verstärkungsteil mit 12, einem oberen Abtriebsteil mit 10 und einem unteren Abtriebsteil mit 9 theoretischen Stufen. Die Kondensation der am Kopf der Kolonne austretenden Dämpfe im Kopfkondensator (K₃C₁) und die teilweise Verdampfung der aus dem unteren Abtriebsteil (K₃AT₂) ablaufenden Flüssigkeit im Verdampfer K₃E₁ für das Sumpfprodukt erfolgt jeweils einstufig.

Die Kolonne wird bei einem Kopfdruck von 15 mbar und einem Rücklaufverhältnis von 0,7 betrieben.

Dabei erhält man als Destillat (2) einen Strom mit 69,9/28,3/1,2/0,5 Gew.-% MPC/Phenol/DMC/DPE. Unterhalb des oberen Verstärkungsteils K₃VT₁ werden 0,02 kg/h Flüssigkeit zwecks Ausschleusung von Zwischensiedern im Seitenstrom (4) entnommen. Ferner werden unterhalb des oberen Verstärkungsteils K₃VT₁ 192,8 kg/h eines dampfförmigen Seitenstroms (6) mit 99,9 Gew.-% DPC entnommen. Als Sumpfprodukt (3) erhält man 20,8 kg/h eines Gemisches mit 69,8/29,6/0,5 Gew.-% DPC/Ti(PhO)4/Salol. Ein Teilstrom des Sumpfproduktes, dessen Menge 10 % bezogen auf die Gesamtmenge des Sumpfproduktes (3) entsprechen, wird einer Rückstandsaufkonzentrierung (K₃RA), bestehend aus einem Dünnschichtverdampfer (K₃E₂), zugeführt. Das restliche Sumpfprodukt (24) wird wieder der zweistufigen Umesterung zur Herstellung des DPC-haltigen Stroms (1) zurückgeführt. Dadurch wird der Katalysatorverlust um 90 % verringert.

Der dampfförmige Seitenstrom (6) wird einer Seitenstromkolonne K₄ zugeführt. Diese verfügt lediglich über einen Verstärkungsteil K₄VT₁ mit 9 theoretischen Stufen.

Die Seitenstromkolonne K₄ wird unter gleichen Druckbedingungen wie die Feindestillation in der ersten Destillationskolonne K₃ und bei einem Rücklaufverhältnis von 0,5 betrieben.

Die am Kopf der Seitenstromkolonne K₄ austretenden Dämpfe (7) werden in einer zweistufigen Kondensation in den Kondensatoren K₄C_{1-N} kondensiert, wobei die Kondensationswärme entweder zur Erzeugung von Dampf oder zur Erwärmung anderer Prozessabschnitte der DPC-Herstellung verwendet wird.

Dabei erhält man ein Destillat mit 99,93 Gew.-% DPC und nur 600 ppm Salol. Die am Sumpf der Seitenstromkolonne ablaufende Flüssigkeit 9 wird der Feindestillation in der ersten Destillationskolonne K₃ oberhalb des unteren Abtriebsteils K₃AT₂ zugeführt.

### Beispiel 2: DPC-Aufreinigung in einer Trennwandkolonne mit flüssiger Seitenstromentnahme

Für dieses Beispiel wird eine Trennwandkolonne gemäß Fig. 3.1 verwendet. Die Trennwandkolonne K₃ besteht aus einem Verstärkungsteil mit 2 Sektionen. Die obere Sektion K₃VT₁ hat eine Trennleistung von 2, die darunter befindliche Sektion K₃VT₂ eine Trennleistung von 12 theoretischen Stufen. Unterhalb des oberen Verstärkungsteils K₃VT₁ besteht die Möglichkeit zur Entnahme eines Flüssigkeitsstroms (4) zur Ausschleusung von Diphenylether.

Unterhalb des Verstärkungsteils wird die Kolonne durch eine vertikal angeordnete Trennwand T partitioniert. Auf der Entnahme- und Zulaufseite dieser Trennwand befinden sich jeweils mindestens 2 Sektionen. Auf der Zulaufseite der Trennwand erfolgt die Aufgabe des Zulaufs (1). Auf der Entnahmeseite erfolgt eine flüssige Entnahme des gereinigten Diphenylcarbonats (5).

Die auf der Zulaufseite der Trennwand befindliche obere Sektion K₃TLO dient zur Abtrennung von Schwersiedern, die im Zulauf (1) enthalten sind. Die untere Sektion der Zulaufseite K₃TLU dient zur Abtrennung von Leichtsiedern, die im Zulauf (1) enthalten sind. Die auf der Entnahmeseite der Trennwand befindliche obere Sektion K₃TRO dient zur Abtrennung von Leichtsiedern, die im, aus dem Verstärkungsteil austretenden, Flüssigkeitsstrom (15) enthalten sind. Die untere Sektion der Entnahmeseite K₃TRU dient zur Abtrennung von Schwersiedern, die im, aus dem Abtriebsteil austretenden, Dampfstrom (17) enthalten sind.

Der Abtriebsteil K₃AT₁ hat eine Trennleistung von 14 theoretischen Stufen und dient zur Aufkonzentrierung der Schwersieder.

Der aus dem Verstärkungsteil austretende Dampf (10) wird in einem Kondensator K₃C_{1-N} kondensiert und teilweise als Rücklauf (11) wieder auf die Kolonne aufgegeben. Die aus dem Abtriebsteil ablaufende Flüssigkeit (12) wird in einem Verdampfer K₃E_{1-N} teilweise verdampft.

Die Kolonne wird bei einem Kopfdruck von 15 mbar betrieben.

Die Zulaufmenge (1) zur Kolonne K₃ beträgt 236,6 kg/h. Darin sind62,8/24,2/9,8/0,4/0,1/0,03/2,6 Gew.-% DPC/MPC/Phenol/DMC/DPE/Salol/Ti(PhO)4 enthalten. Als Destillat (2) erhält man 81,8 kg/h eines Gemisches mit 69,9/28,4/1,15/0,05/0,5 Gew.-% MPC/Phenol/DMC/DPC/DPE. Zur Ausschleusung des DPE werden 0,2 kg/h als Seitenstrom (4) mit 61,3/33,5/0,9 Gew.-% DPC/MPC/DPE aus dem Verstärkungsteil entnommen. Als Sumpfprodukt (3) erhält man 20,6 kg/h einer Mischung aus 69,7/29,8/0,41 Gew.-% DPC/Ti(PhO)4/Salol.

Das im Seitenstrom (5) entnommene Diphenylcarbonat (134 kg/h) hat eine Reinheit von 99,97 Gew.-% und enthält nur 280 ppm Salol.

Die aus dem Verstärkungsteil ablaufende Flüssigkeit wird zu 25 % auf die Zulaufseite der Trennwand, also oberhalb von K₃TLO, und zu 75 % auf die Entnahmeseite der Tennwand, also auf K₃TRO, aufgegeben. Der aus dem Abtriebsteil K₃AT₁ aufsteigende Dampf wird zu gleichen Teilen auf die Zulauf- und Entnahmeseite der Trennwand aufgeteilt.

### Beispiel 3: DPC-Aufreinigung in einer Trennwandkolonne mit dampfförmiger Seitenstromentnahme

Für dieses Beispiel wird eine Trennwandkolonne gemäß Fig. 3.2 verwendet. Die Trennwandkolonne K₃ besteht aus einem Verstärkungsteil mit 2 Sektionen. Die obere Sektion K₃VT₁ hat eine Trennleistung von 2, die darunter befindliche Sektion K₃VT₂ eine Trennleistung von 13 theoretischen Stufen. Unterhalb des oberen Verstärkungsteils K₃VT₁ besteht die Möglichkeit zur Entnahme eines Flüssigkeitsstroms (4) zur Ausschleusung von Diphenylether.

Unterhalb des Verstärkungsteils wird die Kolonne durch eine vertikal angeordnete Trennwand T partitioniert. Auf der Zulauf- und der Entnahmeseite dieser Trennwand befinden sich jeweils mindestens 2 Sektionen. Auf der Zulaufseite der Trennwand erfolgt die Aufgabe des Zulaufs (1). Auf der Entnahmeseite erfolgt eine dampfförmige Entnahme des gereinigten Diphenylcarbonats (6).

Die auf der Zulaufseite der Trennwand befindliche obere Sektion K₃TLO dient zur Abtrennung von Schwersiedern, die im Zulauf 1 enthalten sind. Die untere Sektion der Zulaufseite K₃TLU dient zur Abtrennung von Leichtsiedern, die im Zulauf 1 enthalten sind. Die auf der Entnahmeseite der Trennwand befindliche obere Sektion K₃TRO dient zur Abtrennung von Leichtsiedern, die im, aus dem Verstärkungsteil austretenden, Flüssigkeitsstrom 15 enthalten sind. Die untere Sektion der Entnahmeseite K₃TRU dient zur Abtrennung von Schwersiedern, die im, aus dem Abtriebsteil austretenden, Dampfstrom (17) enthalten sind.

Der Abtriebsteil K₃AT₁ hat eine Trennleistung von 14 theoretischen Stufen und dient zur Aufkonzentrierung der Schwersieder.

Der aus dem Verstärkungsteil austretende Dampf (10) wird in einem Kondensator K₃C_{1-N} kondensiert und teilweise als Rücklauf (11) wieder auf die Kolonne aufgegeben. Die aus dem Abtriebsteil ablaufende Flüssigkeit (12) wird in einem Verdampfer K₃E_{1-N} teilweise verdampft.

Die Kolonne wird bei einem Kopfdruck von 15 mbar betrieben.

Der Kolonne K₃ werden werden 236,6 kg/h mit 62,8/24,2/9,8/0,4/0,1/0,03/2,6 Gew.-% DPC/MPC/Phenol/DMC/DPE/Salol/Ti(PhO)4 als Zulauf (1) zugeführt. Als Destillat (2) erhält man 81,8 kg/h mit 69,8/28,4/1,16/0,1/0,5 Gew.-% MPC/Phenol/DMC/DPC/DPE. Zur Ausschleusung des DPE werden 0,2 kg/h als Seitenstrom (4) mit 68,3/27,1/0,7 Gew.-% DPC/MPC/DPE aus dem Verstärkungsteil entnommen. Als Sumpfprodukt 3 erhält man 20,6 kg/h einer Mischung aus 69,7/29,8/0,41 Gew.-% DPC/Ti(PhO)4/Salol.

Das im Seitenstrom entnommene und anschliessend kondensierte Diphenylcarbonat (5) hat eine Reinheit von 99,97 Gew.-% und enthält nur 290 ppm Salol.

Die aus dem Verstärkungsteil ablaufende Flüssigkeit wird zu 25 % auf die Zulaufseite der Trennwand, also oberhalb von K₃TLO, und zu 75 % auf die Entnahmeseite der Tennwand, also auf K₃TRO, aufgegeben. Der aus dem Abtriebsteil K₃AT₁ aufsteigende Dampf wird zu 25 % auf die Zulauf- und zu 75 % auf Entnahmeseite der Trennwand aufgeteilt.

## Patentansprüche

1. Verfahren zur Reinigung von Diarylcarbonaten in wenigstens einer Destillationskolonne enthaltend wenigstens einen Verstärkungsteil im oberen Teil der Kolonne und wenigstens einen Abtriebsteil im unteren Teil der Kolonne, wobei
• das zu reinigende Diarylcarbonat durch Umesterung aus wenigstens einem Dialkylcarbonat und wenigstens einer aromatischen Hydroxyverbindung in Gegenwart wenigstens eines Umesterungskatalysator hergestellt wurde und Katalysator aus der Herstellung des Diarylcarbonats als Verunreinigung enthält,
• das gereinigte Diarylcarbonat im Seitenstrom der ersten Destillationkolonne entnommen wird, **dadurch gekennzeichnet, dass** es sich bei der ersten Destillationskolonne um eine Trennwandkolonne handelt.

2. Verfahren nach Anspruch 1, **dadurch gekennzeichnet, dass** das im Seitenstrom der ersten Destillationkolonne entnommene Diarylcarbonat in wenigstens einer, bevorzugt einer weiteren Destillationskolonne aufgereinigt wird.

3. Verfahren nach Anspruch 2, **dadurch gekennzeichnet, dass** die weitere Destillationskolonne ohne Abtriebsteil ausgeführt ist.

4. Verfahren nach Anspruch 2 oder 3, **dadurch gekennzeichnet, dass** die weitere Destillationskolonne in die erste Destillationskolonne integriert ist.

5. Verfahren nach wenigstens einem der Ansprüche 1 bis 4, **dadurch gekennzeichnet, dass** das im Seitenstrom der ersten Destillationkolonne entnommene Diarylcarbonat flüssig oder dampfförmig, der ersten Destillationskolonne entnommen wird.

6. Verfahren nach wenigstens einem der Ansprüche 1 bis 5, **dadurch gekennzeichnet, dass** das zu reinigende Diarylcarbonat Verbindungen mit einem Siedepunkt zwischen dem des Diarylcarbonats und dem des während der Herstellung des Diarylcarbonats als Nebenprodukt gebildeten Alkylarylcarbonats als Verunreinigung enthält, welche in einem weiteren Seitenstrom der ersten Destillationskolonne entnommen werden.

7. Verfahren nach Anspruch 6, **dadurch gekennzeichnet, dass** die Verbindungen mit einem Siedepunkt zwischen dem des Diarylcarbonats und dem des während der Herstellung des Diarylcarbonats als Nebenprodukt gebildeten Alkylarylcarbonats in einem Seitenstrom oberhalb der Seitenstomentnahme für das Diarylcarbonat der ersten Destillationskolonne entnommen werden.

8. Verfahren nach wenigstens einem der Ansprüche 1 bis 7, **dadurch gekennzeichnet, dass** der Abtriebsteil der ersten Destillationskolonne in einen unteren und einen oberen Abtriebsteil unterteilt ist.

9. Verfahren nach wenigstens einem der Ansprüche 1 bis 8, **dadurch gekennzeichnet, dass** der Verstärkungsteil der ersten Destillationskolonne in einen unteren und einen oberen Verstärkungsteil unterteilt ist.

10. Verfahren nach wenigstens einem der Ansprüche 1 bis 9, **dadurch gekennzeichnet, dass** die Trennwandkolonne zusätzlich zu wenigstens einem Verstärkungsteil im oberen Teil der Kolonne und wenigstens einem Abtriebsteil im unteren Teil der Kolonne jeweils eine obere und untere Sektion auf der Zulaufseite der Trennwand und auf der Entnahmeseite der Trennwand aufweist, wobei Zulauf und Entnahme jeweils zwischen der oberen und unteren Sektion erfolgen.

11. Verfahren nach wenigstens einem der Ansprüche 1 bis 10, **dadurch gekennzeichnet, dass** die erste Destillationskolonne einen oder mehrere Kopfkondensatoren aufweist, welche in die erste Destillationskolonne integriert sind, wobei das Verhältnis d₁/D₁ von Durchmesser der Dampfleitung von der ersten Destillationskolonne zu dem oder den Kopfkondensator(en) (d₁) zu Kolonnendurchmesser der ersten Destillationskolonne (D₁) im Bereich von 0,2 bis 1 liegt.

12. Verfahren nach wenigstens einem der Ansprüche 2 bis 11, **dadurch gekennzeichnet, dass** wenigstens eine weitere Destillationskolonne einen oder mehrere Kopfkondensatoren aufweist, welche in die weitere Destillationskolonne integriert sind, wobei das Verhältnis d₂/D₂ von Durchmesser der Dampfleitung von der weiteren Destillationskolonne zu dem oder den Kopfkondensator(en) (d₂) zu Kolonnendurchmesser der weiteren Destillationskolonne (D₂) im Bereich von 0,2 bis 1 liegt.

13. Verfahren nach wenigstens einem der Ansprüche 1 bis 12, **dadurch gekennzeichnet, dass** das im Sumpf der ersten Destillationskolonne erhaltene Sumpfprodukt zu mindestens 50 % wieder in die Umesterung aus wenigstens einem Dialkylcarbonat und wenigstens einer aromatischen Hydroxyverbindung zurückführt und das nicht zurückgeführte Sumpfprodukt einer Rückstandsaufkonzentrierung zugeführt wird.

14. Verfahren nach Anspruch 13, **dadurch gekennzeichnet, dass** der bei der Rückstandsaufkonzentrierung anfallende Rückstand einer weiteren Stufe zur Rückgewinnung des Katalysators zugeführt wird.

15. Verfahren nach wenigstens einem der Ansprüche 1 bis 14, **dadurch gekennzeichnet, dass** die Leitungen und Aggregate, die Gemische führen, welche einen Festpunkt von mehr als 30°C aufweisen, auf Temperaturen oberhalb dieses Festpunktes beheizt werden.

## Claims

1. Process for purifying diaryl carbonates in at least one distillation column comprising at least one rectifying section in the upper part of the column and at least one stripping section in the lower part of the column, wherein
• the diaryl carbonate to be purified has been prepared by transesterification from at least one dialkyl carbonate and at least one aromatic hydroxyl compound in the presence of at least one transesterification catalyst and comprises catalyst from the preparation of the diaryl carbonate as an impurity,
• the purified diaryl carbonate is withdrawn in the sidestream of the first distillation column,
**characterized in that** the first distillation column is a dividing wall column.

2. Process according to Claim 1, **characterized in that** the diaryl carbonate withdrawn in the sidestream of the first distillation column is purified in at least one distillation column, preferably in one further distillation column.

3. Process according to Claim 2, **characterized in that** the further distillation column is designed without a stripping section.

4. Process according to Claim 2 or 3, **characterized in that** the further distillation column is integrated into the first distillation column.

5. Process according to at least one of Claims 1 to 4, **characterized in that** the diaryl carbonate withdrawn in the sidestream of the first distillation column is withdrawn from the first distillation column in liquid or vaporous form.

6. Process according to at least one of Claims 1 to 5, **characterized in that** the diaryl carbonate to be purified comprises compounds having a boiling point between that of the diaryl carbonate and that of the alkyl aryl carbonate formed as a by-product during the preparation of the diaryl carbonate as an impurity, which is withdrawn in a further sidestream of the first distillation column.

7. Process according to Claim 6, **characterized in that** the compounds having a boiling point between that of the diaryl carbonate and that of the alkyl aryl carbonate formed as a by-product during the preparation of the diaryl carbonate are withdrawn from the first distillation column in a sidestream above the sidestream withdrawal for the diaryl carbonate.

8. Process according to at least one of Claims 1 to 7, **characterized in that** the stripping section of the first distillation column is divided into a lower stripping section and an upper stripping section.

9. Process according to at least one of Claims 1 to 8, **characterized in that** the rectifying section of the first distillation column is divided into a lower rectifying section and an upper rectifying section.

10. Process according to at least one of Claims 1 to 9, **characterized in that** the dividing wall column, in addition to at least one rectifying section in the upper part of the column and at least one stripping section in the lower part of the column, has in each case an upper and lower section on the feed side of the dividing wall and on the withdrawal side of the dividing wall, feed and withdrawal each being effected between the upper and lower section.

11. Process according to at least one of Claims 1 to 10, **characterized in that** the first distillation column has one or more top condensers which are integrated into the first distillation column, where the ratio d₁/D₁ of diameter of the vapour line from the first distillation column to the top condenser(s) (d₁) to column diameter of the first distillation column (D₁) is in the range of 0.2 to 1.

12. Process according to at least one of Claims 2 to 11, **characterized in that** at least one further distillation column has one or more top condensers which are integrated into the further distillation column, where the ratio d₂/D₂ of diameter of the vapour line from the further distillation column to the top condenser(s) (d₂) to column diameter of the further distillation column (D₂) is in the range of 0.2 to 1.

13. Process according to at least one of Claims 1 to 12, **characterized in that** the bottom product obtained in the bottom of the first distillation column is recycled to an extent of at least 50% back into the transesterification from at least one dialkyl carbonate and at least one aromatic hydroxyl compound, and the unrecycled bottom product is sent to a residue concentration.

14. Process according to Claim 13, **characterized in that** the residue obtained in the residue concentration is sent to a further stage for recovery of the catalyst.

15. Process according to at least one of Claims 1 to 14, **characterized in that** the lines and units which conduct mixtures which have a melting point of more than 30°C are heated to temperatures above this melting point.

## Revendications

1. Procédé pour la purification de carbonates de diaryle dans au moins une colonne de distillation contenant au moins une partie de concentration dans la partie supérieure de la colonne et au moins une partie d'épuisement dans la partie inférieure de la colonne,
- le carbonate de diaryle à purifier ayant été préparé par transestérification à partir d'au moins un carbonate de dialkyle et d'au moins un composé hydroxy aromatique en présence d'au moins un catalyseur de transestérification et contenant le catalyseur de la préparation du carbonate de diaryle comme impureté,
- le carbonate de diaryle purifié étant prélevé dans le flux latéral de la première colonne de distillation.
**caractérisé en ce qu'**il s'agit, pour la première colonne de distillation, d'une colonne à paroi de séparation.

2. Procédé selon la revendication 1, **caractérisé en ce que** le carbonate de diaryle prélevé dans le flux latéral de la première colonne de distillation est purifié dans au moins une, de préférence une autre, colonne de distillation.

3. Procédé selon la revendication 2, **caractérisé en ce que** l'autre colonne de distillation est réalisée sans partie d'épuisement.

4. Procédé selon la revendication 2 ou 3, **caractérisé en ce que** l'autre colonne de distillation est intégrée dans la première colonne de distillation.

5. Procédé selon au moins l'une quelconque des revendications 1 à 4, **caractérisé en ce que** le carbonate de diaryle prélevé dans le flux latéral de la première colonne de distillation est prélevé sous forme liquide ou de vapeur de la première colonne de distillation.

6. Procédé selon au moins l'une quelconque des revendications 1 à 5, **caractérisé en ce que** le carbonate de diaryle à purifier contient, comme impuretés, des composés présentant un point d'ébullition entre celui du carbonate de diaryle et celui du carbonate d'alkylaryle formé comme produit secondaire lors de la préparation du carbonate de diaryle, qui sont prélevés dans un autre flux latéral de la première colonne de distillation.

7. Procédé selon la revendication 6, **caractérisé en ce que** les composés présentant un point d'ébullition entre celui du carbonate de diaryle et celui du carbonate d'alkylaryle formé comme produit secondaire lors de la préparation du carbonate de diaryle sont prélevés dans un flux latéral au-dessus du prélèvement de flux latéral pour le carbonate de diaryle de la première colonne de distillation.

8. Procédé selon au moins l'une quelconque des revendications 1 à 7, **caractérisé en ce que** la partie d'épuisement de la première colonne de distillation est divisée en une partie d'épuisement inférieure et une partie d'épuisement supérieure.

9. Procédé selon au moins l'une quelconque des revendications 1 à 8, **caractérisé en ce que** la partie de concentration de la première colonne de distillation est divisée en une partie de concentration inférieure et une partie de concentration supérieure.

10. Procédé selon au moins l'une quelconque des revendications 1 à 9, **caractérisé en ce que** la colonne à paroi de séparation présente, en plus d'au moins une partie de concentration dans la partie supérieure de la colonne et d'au moins une partie d'épuisement dans la partie inférieure de la colonne, à chaque fois une section supérieure et une section inférieure sur le côté alimentation de la paroi de séparation et sur le côté prélèvement de la paroi de séparation, l'alimentation et le prélèvement ayant à chaque fois lieu entre la section supérieure et la section inférieure.

11. Procédé selon au moins l'une quelconque des revendications 1 à 10, **caractérisé en ce que** la première colonne de distillation présente un ou plusieurs condenseurs de tête, qui sont intégrés dans la première colonne de distillation, le rapport d₁/D₁ du diamètre de la conduite de vapeur de la première colonne de distillation vers le ou les condenseur(s) de tête (d₁) au diamètre de la première colonne de distillation (D₁) se situant dans la plage de 0,2 à 1.

12. Procédé selon au moins l'une quelconque des revendications 2 à 11, **caractérisé en ce qu'**au moins une autre colonne de distillation présente un ou plusieurs condenseurs de tête, qui sont intégrés dans l'autre colonne de distillation, le rapport d₂/D₂ du diamètre de la conduite de vapeur de l'autre colonne de distillation vers le ou les condenseur(s) de tête (d₂) au diamètre de l'autre colonne de distillation (D₂) se situant dans la plage de 0,2 à 1.

13. Procédé selon au moins l'une quelconque des revendications 1 à 12, **caractérisé en ce que** le produit du fond, obtenu dans le fond de la première colonne de distillation, est recyclé à raison d'au moins 50% dans la transestérification d'au moins un carbonate de dialkyle et d'au moins un composé hydroxy aromatique et le produit de fond non recyclé est introduit dans une concentration de résidu.

14. Procédé selon la revendication 13, **caractérisé en ce que** le résidu produit lors de la concentration de résidu est introduit dans une autre étape pour la récupération du catalyseur.

15. Procédé selon au moins l'une quelconque des revendications 1 à 14, **caractérisé en ce que** les conduites et les appareils, qui guident les mélanges, qui présentent un point de solidification supérieur à 30°C, sont chauffés à des températures supérieures à ce point de solidification.
